# EUROPEAN PATENT SPECIFICATION

(11) **EP 0 759 992 B1**
(45) Date of publication and mention of the grant of the patent: **27.10.2004**
(21) Application number: 95920437.1
(22) Date of filing: 15.05.1995
(51) Int. Cl.: C12N 15/52, C12N 9/00, A61K 31/70, C12Q 1/68

(54) **HUMAN PAPILLOMA VIRUS INHIBITION BY A HAIRPIN RIBOZYME**
HEMMUNG VON MENSCHLICHEM PAPILLOMAVIRUS DURCH "HAIRPIN-RIBOZYME"
INHIBITION DU PAPILLOMAVIRUS HUMAIN A L'AIDE D'UN RIBOZYME EN FORME D'EPINGLE A CHEVEUX

(30) Priority: 13.05.1994 US 242665
(43) Date of publication of application: 05.03.1997
(60) Divisional application: 03075150.7
(73) Proprietor: THE GOVERNMENT OF THE UNITED STATES OF AMERICA, as represented by THE SECRETARY, DEPARTMENT OF HEALTH AND HUMAN SERVICES, Rockville, MD 20852 (US); THE BOARD OF REGENTS FOR NORTHERN ILLINOIS UNIVERSITY, DeKalb, Illinois 60115 (US)
(72) Inventor: HAMPEL, Arnold, DeKalb, IL 60115 (US); DIPAOLO, Joseph, Bethesda, MD 20817 (US); SIWKOWSKI, Andrew, Sycamore, IL 60178 (US)
(74) Representative: Van Malderen, Michel
(86) International application number: PCT/US1995/006016
(87) International publication number: WO 1995/031552

(56) References cited:
- WO-A-93/23569
- 83RD ANNUAL MEETING OF THE AMERICAN ASSOCIATION FOR CANCER RESEARCH, SAN DIEGO, CALIFORNIA, USA, MAY 20-23, 1992. & PROC AM ASSOC CANCER RES ANNU MEET 33 (0). 1992. 307, EVANS, C. ET AL. 'REGULATION OF HUMAN PAPILLOMA VIRUS 16 HPV16 DNA POSITIVE CERVICAL EPITHELIAL CELL SENSITIVITY TO LAK LYMPHOCYTE LYSIS BY TREATMENT WITH CISPLATIN AND THE CYTOKINES GAMMA INTERFERON OR LEUKOREGULIN.'
- NATURE, vol. 346, 16 August 1990 LONDON GB, pages 680-682, CHEONG, C. ET AL. 'Solution structure of an unusually stable RNA hairpin, 5'GGAC(UUCG)GUCC' cited in the application
- FEBS (FED EUR BIOCHEM SOC) LETT 322 (1). 1993. 21-24, HE, Y.-K. ET AL. 'IN-VITRO CLEAVAGE OF HPV16 E6 AND E7 RNA FRAGMENTS BY SYNTHETIC RIBOZYMES AND TRANSCRIBED RIBOZYMES FROM RNA-TRIMMING PLASMIDS.'

## Description

### Field of the Invention

The present invention relates to an RNA catalyst, i.e., ribozyme, which cleaves Human Papilloma virus into a fragment having a 5' hydroxyl and a fragment having a 2',3' cyclic phosphate. The products of the reaction described herein resemble those resulting from the natural hydrolysis of RNA.

### Background of the Invention

Papilloma viruses are small DNA viruses that induce the hyperproliferation of epithelial cells. Approximately 70 different genotypes have been isolated from humans. Some types (1, 2, 4, and 7) are associated with benign squamous papillomas (warts; condylomas) in humans, while at least two types (16 and 18) have been associated with human neoplastic and preneoplastic lesions (DiPaolo, et al., 1993, Critical Reviews in Oncogenesis 4:337-360).

In the United States, cervical cancer affects approximately 8.6 women per 100,000 each year. In women, HPV·16 is frequently associated with latent infections, benign and premalignant cervical lesions (dysplasias/CIN) and half of invasive cervical carcinomas. In males, HPV-16 is associated with subclinical macular or clinical papular lesions. Bowenoid papulosis of the penis resembles carcinoma *in situ*. Cervical cancer, which kills at least 500,000 women worldwide each year, proceeds through progressive cellular changes from benign condylomata to high-grade dysplasias /CIN before developing into an invasive cancer. Over five billion health care dollars are spent in the United States each year on the detection and treatment of these lesions.

Epidemiology evidence indicates that up to 89% of all human and oral tumors harbor types of HPV that are able to immortalize primary human keratinocytes and transform rodent cells. The oncogene potential of HPV appears to be associated with products from two viral genes, E6 and E7. These products are required for the acquisition and maintenance of a transformed phenotype. The proteins encoded by these genes bind, with high affinity in neoplastic·associated types, to and neutralize the products of the *Rb* and p53 tumor suppressor cells (Nasseri, 1991, Virol. 194:136; Sedman, et al., 1991, J. Virol. 65:4860-4866; Smotkin and Wettstein, 1989, J. Virol. 63:1441-1447; Smotkin and Wettstein, 1986, J. Virol. 63:1441-1447; Steele, et al., 1993, Cancer Res. 53:2330; Storey, et al., 1991, Nuc. Acids Res. 19(15):4109).

The current policy in genitourinary clinics is surgery for high-grade lesions due to the lack of superior alternatives. Cervical laser ablation therapy does not in the long term influence the natural history of cervical human papillomavirus-associated diseases in women. Interferons, *per se,* have been disappointing insofar as acute viral infection is concerned, usually because treatment cannot be started in time. Therefore, it has been assumed that any benefit with interferons is due to antiproliferative effect and not due to antiviral.

Combination chemotherapy is also in use in cancer therapy, and cisplatin is one of the drugs of choice for cervical cancer, alone or in combination with other chemotherapy agents. However, the current success obtained with chemotherapy treatment is poor. The response rate for combination cisplatin and 5FU treatment in phase II studies in cervical cancer patients is only effective in 22% of the patients while the same combination produced an 88% response in squamous cell carcinoma of the head and neck.

The use of cytotoxic agents for cancer therapy has limitations because of toxic side effects and the development of multiple drug resistance. Therefore, there has been a consideration of a shift to therapy which does not involve direct toxic reaction, but which can modify the growth of tumor cells.

Current new therapeutic suggestions for treatment of HPV infections have centered on the use of antisense oligonucleotides to interrupt viral mRNA utilization (DiPaolo, et al., 1993, Critical Reviews in Oncogenesis 4:337-360; Steele, et al., 1993, Cancer Res. 53:2330; Storey, et al., 1991, Nuc. Acids Res. 19(15):4109). However, antisense therapy is limited by stoichiometric considerations (Sarver, et al., 1990, Gene Regulation and Aids pp. 305-325).

Ribozymes are RNA molecules that possess RNA catalytic ability (see Cech, et al., U.S. Patent 4,987,071) that cleave a specific site in a target RNA. The number of RNA molecules that are cleaved by a ribozyme is greater than the number predicted by stochiochemistry (Hampel and Tritz, 1989, Biochem. 28:4929-4933; Uhlenbeck, 1987, Nature 328:596.600). This provides an advantage over the antisense technology.

Antisense therapy has two disadvantages when compared to ribozymes: (1) by its nature, the antisense molecule is not catalytic; and (2) antisense molecules are normally longer than the ribozyme target recognition sequence. This increases the likelihood of antisense molecules having a deleterious effect on similar mRNA sequences found in the same gene family.

Ribozymes have been designed on the "hammerhead" motif (Sedman, et al., 1991, J. Virol. 65:4860-4866). However, catalytic RNAs such as those that were designed based on the "hammerhead" model have several limitations which restrict their use *in vitro* and may forestall their use *in vivo.* For example, the temperature optimum for the reaction is 50-55°C (Haseloff and Gerlach, 1988, Nature 334:585; Uhlenbeck, 1987, Nature 328:596-600). In addition, the Km is 0.6µM (Uhlenbeck, 1987, Nature 328:596-600), meaning that the reaction requires high concentrations of substrate which makes it difficult, if not impossible, for the catalytic RNA to cleave low levels of target RNA substrate such as would be encountered *in vivo.*

A "hairpin" motif has been found to be more efficient than the "hammerhead" motif (Hampel and Tritz, 1989, Biochem. 28:4929-4933; Hampel, et al, 1990, Nuc. Acids Res. 18:299-304). Further, hairpin ribozymes have been used to cleave targets on HIV (Ojwang, et al., 1992, Proc. Natl Acad. Sci. USA 89, 10802-10806; Yu, et al., 1993, Proc. Natl. Acad. Sci. USA 90:6340-6344). However, ribozymes for one virus generally will not cleave other virus species. Not only do the ribozymes require specific target sequences for cleavage, they require modifications in the ribozyme structure itself to be able to efficiently deave a specific target. Currently, there is no hairpin ribozyme that has been shown to cleave HPV RNA and no site has been identified in the HPV that is capable of cleavage by a hairpin ribozyme.

Certain molecular components have been used in the past to deliver ribozymes. For example, retroviral vectors have been used to deliver ribozymes. Pollll promoters and retroviral vectors with PoIIII promoters have also been used.

### Summary of the Invention and Advantages

According to the present invention, synthetic catalytic RNAs, i.e., ribozymes, including a hairpin portion, a binding site for binding to a human papilloma virus after viral base 434 and a cleavage site for cleaving the virus at the binding site have been constructed.

In one aspect, the present invention comprises a synthetic ribozyme comprising a hairpin portion, a binding site for binding to a nucleotide sequence of a human papilloma virus, and a cleavage site for cleaving the sequence. The cleavage site is a site after base 434 of the viral sequence of the viral sequence. The binding site of such a synthetic ribozyme preferably binds to one of the following sequences. 430-ACUG U*GUC CUGAAGA-444 (SEQ ID NO:2), 430-ACUG U*GUC CUGAAGAA-445 (SEQ ID NO:3), 430-ACUG U*GUC CUGAAGAAA-446 (SEQ ID NO:4), "*" indicates the cleavage site. The hairpin portion of the ribozyme also preferably comprises the sequence of SEQ ID NO:5.

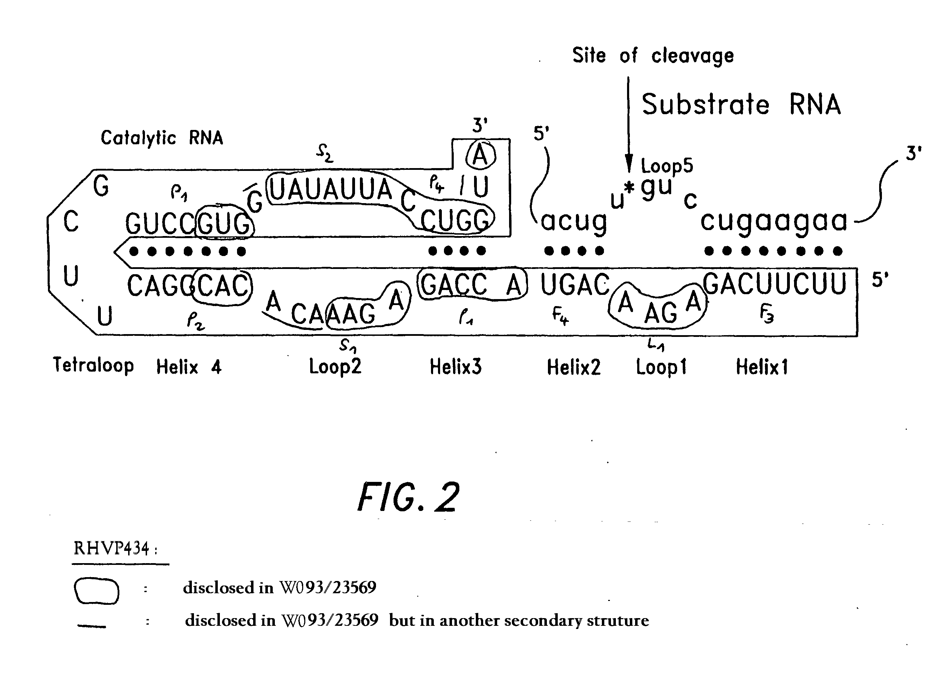

In a preferred embodiment, the synthetic ribozyme binds to a nucleotide sequence of HPV-16. In this embodiment, the ribozyme preferably comprises the sequence of SEQ ID NO:6 . In a further embodiment, the synthetic ribozyme can have the two dimensional configuration shown in Figure 2.

In another aspect of the present invention, the invention includes a vector comprising a DNA sequence coding for the synthetic ribozyme described above. In this aspect, the DNA sequence is preferably operatively linked to expression control sequences. The vector can be, for example, a plasmid. A further aspect of the invention comprises a host cell transformed with the vector described above, wherein the host cell is capable of expressing the ribozyme from the vector.

In yet another aspect of the present invention, the invention comprises a method of cleaving a human papilloma virus by means of a ribozyme. This method includes the steps of:
identifying a cleavage site on the genome of the virus;
determining the sequence on either side of the cleavage site;
constructing a hairpin synthetic ribozyme wherein a binding site on the synthetic ribozyme includes a sequence noncomplementary to the cleavage site and a binding region complementary to the sequences on either side of the cleavage site; and
providing the hairpin synthetic ribozyme to the viral genome, thereby allowing the ribozyme to cleave the viral genome.

In this aspect of the invention, the providing step can be performed *in vitro.* Alternatively, the providing step can be performad *in vivo.* The step of constructing a hairpin synthetic ribozyme can also further include incorporating the tetraloop sequence of SEQ ID NO:5 into the ribozyme.

In a further aspect of the present invention, the invention comprises a method for detecting a human papilloma virus-16 (HPV-16) in human tissue. This method comprises the steps of:
obtaining a sample of human tissue containing RNA;
exposing the RNA in the tissue to a ribozyme which binds to a nucleotide sequence from HPV.16 RNA, such that HPV-16 RNA present in the sample is cleaved by the ribozyme;
amplifying the cDNA using primers complementary to: a 5' end of a full-length HPV-16 transcript, a fragment 5' of the ribozyme cleavage site of the full-length HPV-16 transcript, and a fragment 3' of the ribozyme cleavage site of the full-length HPV-16 transcript; and
identifying DNA fragments amplified.

In the foregoing method, a larger DNA fragment represents a full-length HPV transcript and a smaller sized DNA fragment represents the fragment resulting from ribozyme cleavage of the full-length HPV transcript. If HPV-16 RNA is present in the sample, a preponderance of the smaller fragment is identified relative to the larger fragment. In one embodiment, the HPV-16 RNA cleaved by the ribozyme is an E6 transcript of HPV-16. The ribozyme used in this method is preferably either RHPV434, and the human tissue is a sample of cervical tissue. This method can additionally comprise the step of producing cDNA from the RNA present in the sample after the exposing step and before the amplifying step.

Another aspect of the present invention comprises a method of treating cervical cancer, comprising the steps of:
constructing a synthetic rihozyme comprising a hairpin portion, a binding site for binding to a nucleotide sequence of a human papilloma virus, and a cleavage site for cleaving the sequence, wherein the cleavage site is selected from the group consisting of a site after base 434 of the sequence ; and
delivering an effective amount of the synthetic ribozyme to cervical tissue.

In this method, the step of delivering can comprise suspending the synthetic ribozyme in a lipofection-based liposomal delivery system. In addition, this method can further comprise the step of administering additional agents in combination with the synthetic ribozyme, such as immunological agents or chemotherapeutic agents. Preferably, the immunological agents are LAK cells and the chemotherapeutic agent used is cisplatin.

### Brief Description of the Drawings

Other advantages of the present invention will be readily appreciated as the same becomes better understood by reference to the following detailed description when considered in connection with the accompanying drawings.
FIGURE 1 is a diagram of the HPV target sites, showing the location of the target sites selected for cleavage by the hairpin ribozyme, the overlaps in the target sites of the mRNA for both the E6 and E7 region of HPV16, and the cleavage point of this target site by the hairpin ribozyme which occurs at the "*" after nucleotide 434 and nucleotide 419.
FIGURE 2 is a diagram of the hairpin ribozyme with an optimized helix 1, comprising 8 bp, designed to cleave HPV-16 after position 434, showing the sequences of the optimized ribozyme (RHPV) and the substrate (SHPV), the regions of base pairing between target substrate and riboryme (labeled Helix 1 and Helix 2), and the regions of base pairing required in the "hairpin" portion of the catalyst (labeted Helices 3 and 4).
FIGURE 3 is an autoradiograph of the results of cleavage of HPV substrates after the 434 site by the present invention with helix 1 having lengths of 7 bp, 8 bp, and 9 bp as shown; reference controls were ribozyme R53 and substrate S17 (lanes 1 and 2), the reaction was at 37°C with 25 nM ribozyme and 50 nM substrate for 60 minutes, and the reference reaction was native (-)sTRSV sequence S17/R53 at 10 nM and 100 nM for the times shown (Hampel and Tritz, 1989, Biochem. 28:4929-4933).
FIGURE 4 illustrates the results of a time course of the cleavage by RHPV434 showing: (FIG. 4A) an autoradiograph of the cleavage at each time point, and (FIG. 4B) a graph of the results from 4A, wherein the cleavage conditions were the same as in FIGURE 3 using [R]=25 nM and [S]=100 nM for the times shown.
FIGURE 5 illustrates the kinetic analysis of cleavage by RHPV434, showing: (FIG. 5A) a graph of the results from FIG. 4B, and (FiG. 5B) an autoradiograph of the cleavage results after 10 minutes at each concentration of [S], with cleavage conditions as in FIGURE 3 using [R]=20 nM and [S] of 400 nM (lane 1), 200 nM (lane 2), 150 nM (lane 3), 100 nM (lane 4), 75 nM (lane 5), 50 nM (lane 6), and 25 nM (lane 7).
FIG. 6 shows diagrammatically the polylinker region that was cloned into pBluescript KS to give the plasmid pBKS LNKR.
FIG. 7 is a diagram of the plasmid ptV1 which contains the tRNA^{val} promoter, its upstream region, tetraloop variant and polyT termination sequence. This was prepared by cloning the tRNA^{val} promoter and its upstream region into pBKS LNKR.
FIG. 8 shows the DNA sequence (SEQ ID NO: 19) of plasmid ptV1 including the polylinker, tRNA^{val} promoter, upstream region, tetraloop, termination region; new ribozymes can be cloned into the Xho1/Mlu1 sites.
FIG. 9 is a diagram of the plasmid pBtV1-434 which contains the RHPV434 ribozyme in plasmid ptV1.
FIG. 10 shows the DNA sequence (SEQ ID NO: 20) of the polylinker and ribozyme in the plasmid pBtV1-434.
FIG. 11 is a diagram of the plasmid pZIPV1-434(syn) which contains the RHPV434 ribozyme in the "syn" orientation in which transcription is the same direction as that of the retroviral genome in the retroviral vector.
FIG. 12 shows the DNA sequence (SEQ ID NO: 21) of the delivery cassette and ribozyme in the plasmid pZIP V1-434(syn).
FIG. 13 is a diagram of the plasmid pZIPV1-434(anti) which contains the RHPV434 ribozyme in the "anti" orientation in which transcription is in the opposite direction as that of the retroviral genome in the retroviral vector.
FIG. 14 shows the DNA sequence (SEQ ID NO: 22) of the polylinker and ribozyme in the plasmid pZIP V1-434(anti).
FIG. 15 is a diagram of the plasmid pB1V1-434(i) which contains the catalytically inactive mutant RHPV434 ribozyme clone into ptV1.
FIG. 16 shows the DNA sequence (SEQ ID NO: 23) of the delivery cassette and ribozyme in the plasmid pBtV1-434(i).
FIG. 17 shows expression of the HPV specific ribozyme in transformed human CXT1 carcinoma cells as assayed by RNase protection of transcripts; the arrow indicates the expected size of the protected ribozyme transcript from the pollll promoter, 160 nt.
FIG. 18 shows expression of the HPV E6 transcript in CXT1 cells containing the hairpin ribozyme as assayed by the RT and PCR method.

### Detailed Description of the Invention

A hairpin ribozyme containing a tetraloop modification was designed, tested and shown to cleave a specific sequence in the primary transcript from human papilloma virus type 16. The cleavage sites immediately followed nucleotide 434 , in the sequence of this virus. Optimization of the ribozyme was carried out showing that an 8 nt helix 1 was optimal for the 434 site. The time course of the reaction showed nearly complete cleavage of the substrate.

Kinetic parameters for the 434 site were measured using standard Michaelis enzyme kinetic analysis. The Km for the reaction was 21 nM which shows very tight binding of the ribozyme and substrate. The kcat or turnover number was 0.083 min⁻¹ to give an overall catalytic efficiency (kcat/Km) of 4µM⁻¹ min⁻¹.

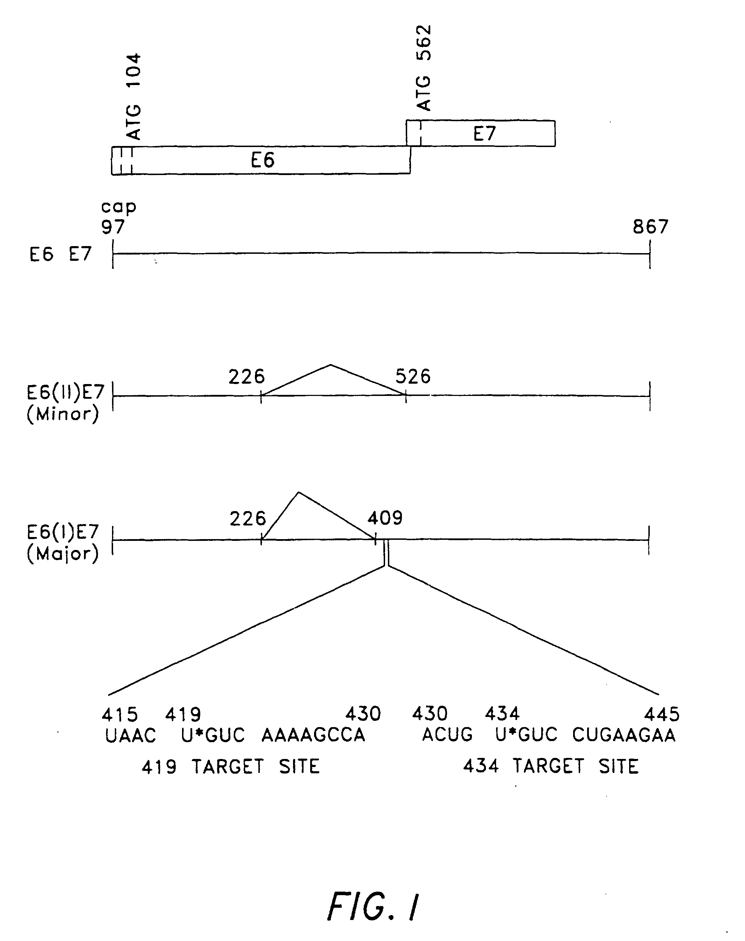

The optimized target sites are shown in Figure 1. Cleavage occurred after base 434 and after base 419, respectively, and before the GUC sequence shown as indicated in the diagram. This entire target sequence is part of the primary transcript (SEQ ID No:1) for the E6 and E7 regions of HPV16 (Nasseri, 1991, Virol. 194:136; Smotkin and Wettstein, 1989, J. Virol. 63:1441.1447). The cap for this mRNA is on nt 97. A splice donor exists at nt 226, and two splice acceptors exist at nt 409 and nt 526. As a result, three different E6-E7 mRNAs can be produced: E6E7, E6:(I)E7, and E6(II)E7. E6E7 is the result of the full-length E6E7 transcript, in which the splice donor at nt 226 is not utilized. In E6E7, translation termination of E6 occurs at nt 557. E6(I)E7, the major transcript, is the result of utilization of the splice donor at nt 226 and the splice acceptor at nt 409, and its E6(I) translation termination signal is at nt 415. This gives a truncated E6 coding region and a full-length E7. E6(II)E7, the minor transcript, is the result of utilization of the splice donor at nt 226 and the splice acceptor at nt 526, and its E6(II) translation termination signal is at nt 541 to give a truncated E6 coding region and a full-length E7 (Nasseri, 1991, Virol. 194:136).

An RNA catalyst (ribozyme) has been identified comprising an RNA sequence which can cleave, with great precision, HPV. The target sequences for cleavage by the rihozymes is present in the primary transcript E6E7, and E6(I)E7, the major transcript. Cleavage of these transcripts would have the effect of lowering the production of full-length E6 and E7 proteins, both of which appear to play a key role in keratinocyte transformation (Sedman, et al., 1991, J. Virol. 65:4860-4866).

The hairpin ribozyme (Hampel, et al., 1990, Nuc. Acids Res. 18:299-304) designed to cleave after the 434 site in HPV is shown in Figure 2 and is designated RHPV434. In the preferred embodiment, this hairpin ribozyme has the tetraloop modification as shown (Anderson, et aL, 1994, Nuc. Acids Res. 22:1096-1100). The GUU sequence of Loop 3 of the basic structure has been replaced by a tetraloop sequence GGAC (UUCG) GUCC which in the present invention has been shown to generate a very stable structure with high catalytic efficiency. In particular, the invention comprises certain synthetic RNA catalysts capable. of cleaving an RNA substrate which contains the target sequences:

"Synthetic RNA catalyst," as used herein, means a catalyst (ribozyme) which is not a naturally-occurring RNA catalyst, although "synthetic catalysts" can be truncated or altered versions of naturally. occurring catalysts. "Synthetic catalyst" include catalysts synthesized *in vitro* and catalysts synthesized *in vivo*. In particular, "synthetic catalysts" can include catalysts produced by hosts transformed by a vector comprising a sequence coding for the catalyst

RNA of any length and type can be used as the substrate as long as it contains the target sequence represented by the formula 5'-F₁-CS-F₂-3'. In this formula, CS is the cleavage sequence, i.e., a sequence of bases containing the site at which the catalyst cleaves the substrate. CS is a short sequence of bases which does not base pair with the ribozyme, and in the present invention CS preferably has the sequence 5'-NGUC-3', wherein N is any base, and the substrate is cleaved by the ribozyme between N and G to produce a fragment having an OH at the 5' end and a fragment having a 2,'3' cyclic phosphate at the 3' end.

CS is flanked by two short base sequences F₁ and F₂ which do base pair with the RNA catalyst. F₁ is preferably at least 3 bases in length, most preferably 4 bases in length. F₂ is also preferably at least three bases in length, most preferably 6 to 12 bases in length.

Rihozymes, according to the present invention, also include a substrate binding portion and a "hairpin" portion. The substrate binding portion of the catalyst is represented by the following formula:

3'F₄-L₁-F₃-5'

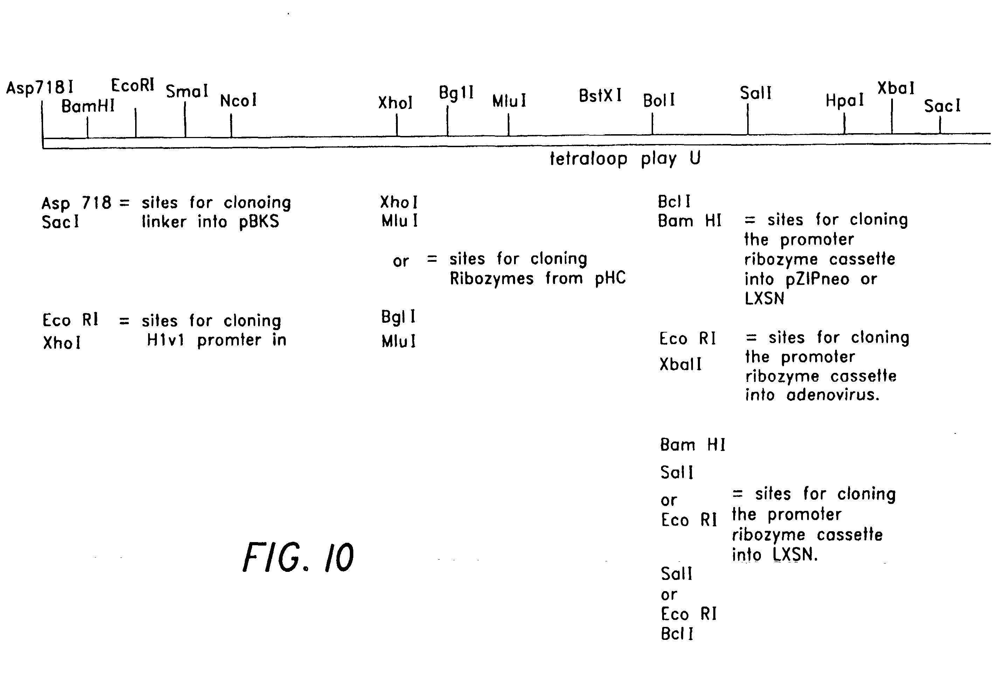

wherein,
F₃ is a sequence of bases selected so that F₃ is substantially base paired with F₂ (Helix 1, Figures 2 and 6) when the catalyst is bound to the substrate;
F₄ is a sequence of bases selected so that F₄ is substantially base paired with F₁ when the catalyst is bound to the substrate (Helix 2, Figures 2),

The sequences of F₃ and F₄ are selected so that each contains an adequate number of bases to achieve sufficient binding of the RNA substrate to the RNA catalyst so that cleavage of the substrate can take place; and
L₁ is a sequence of bases selected so that L₁ does not base pair with CS when the catalyst is bound to the substrate (Loop 1, Figures 2).

As used herein, "substantially base paired" means that greater than 65% of the bases of the two RNA sequences in question are base paired, and preferably greater than 75% of the bases are base paired. "Substantially unpaired" means that greater than 65% of the bases of the two sequences in question are not base paired, and preferably greater than 75% of the bases are not paired.

F₃ is preferably at least 3 bases in length, most preferably from 6 to 12 bases in length. F₄ is preferably from 3 to 5 bases in length, most preferably 4 bases in length.

L₁ is a short sequence of bases which preferably has the sequence 5'-AGAA-3' when CS has the sequence 5'-NGUC-3'. Further, when L₁ is 5'-AGAA-3' and CS is 5'-NGUC-3', then the first base pair between F₁ and F₄ adjacent to CS and L₁ is preferably 6:C or C:G (Figures 2)). Accordingly, in the present invention a preferred target sequence in a selected substrate contains the sequence 5'-BNGUC-3', wherein B is G, C, or U (Anderson, et al., 1994, Nuc. Acids Res. 22:1096-1100).

The "hairpin" portion is a portion of the catalyst which folds into a hairpin-like configuration when the substrate-catalyst complex is modeled in two dimensions for minimum energy folding. This is shown in Figures 2) The "hairpin" portion is not an absolute hairpin in the sense that not all bases of the "hairpin" portion are base-paired. Indeed, it is necessary for the "hairpin" portion to have at least one substantially unpaired region so that the catalyst can assume a tertiary structure that allows for better, or optimal, catalytic activity.

The "hairpin" portion of the catalyst preferably has the sequence: wherein,
P₁ and P₄ are base sequences selected so that P and P₄ are substantially base paired (Helix 3, Figures 2 and 6).
P₁ is covalently attached to F₄;
S₁ and S₂ are sequences selected so that S₁ (Loop 2) and S₂ (Loop 4) are substantially unpaired;
P₂ and P₃ are base sequences selected so that P₂ and P₃ are substantially base paired (Helix 4, Figures 2 and 6); and
L₂ is a sequence of unpaired bases (Loop 3).

"Substantially base paired'' and "substantially unpaired'' have the same meanings as discussed above.

P₁ and P₄ are each preferably from 3 to 6 bases in length, and most preferably P₁ has the sequence 5'-ACCAG-3' and P₄ has the sequence 5'-CUGGUA-3'. It has been found that the A at the 5' end of 5'-ACCAG-3' (underlined) is not base paired to the U at the 3' end of 5'-CUGGUA-3' (underlined), and the unpaired A may act as a "hinge" (Figures 2).

S₁ and S₂ are each preferably from 4 to 9 bases in length, and most preferably S₁ has the sequence 5'-AGAAACA-3' and S₂ has the sequence 5'-GUAUAUUAC-3'.

Unexpectedly, it was found that the hairpin ribozyme as constructed for an HIV target sequence (Ojwang, et al., 1992, Proc. Natl. Acad. Sci. USA 89, 10802-10806) was not as efficient as a hairpin ribozyme constructed with a "tetraloop" modification.

In the prior art the preferred sequence P₂ has the sequence 5'-CAC-3', P₃ has the sequence 5'-GUG-3' and L₂ has the sequence 5'-GUU-3'(Ojwang, et al., 1992, Proc. Natl. Acad. Sci. USA 89, 10802-10806).

In the preferred embodiment of the present invention L₂, P₂, P₃ (Figures 2) 6, Loop 3, Helix 4). are constructed to include the stable RNA hairpin sequence.

5'-GGAC UUCG GUCC -3' (SEQ ID NO:5) results in the "tetraloop" modification. As a result, Helix 4 is extended by four base pairs over the prior art sequence listed hereinabove. Further, the GUU sequence of Loop 3 is replaced with the sequence UUCG. The resulting ribozyme is more active and more thermally stable than the non-modified ribozyme.

The structure of the present invention as shown in Figure 2 for RHVP434. and described hereinabove can be diagrammatically represented by the formula: The complete sequence of the ribozyme of the preferred embodiment of the present invention is 5'-UUCUUCAGAGAACAGU ACCAGAGAAACACACGGACUUCGUCCGUGGUAUAUUACCUGGUA-3' (SEQ ID No:6).

The ribozyme of the present invention which cleaves the RNA of HPV can be used as a therapeutic agent in the treatment of HPV infections which are associated with genital warts and genital neoplasms.

In the preferred embodiment, there are two methods for administering the therapeutic agent: gene therapy and a modification of antisense methodology. The therapeutic agent utilized in the present invention is administered in combination with other drugs or singly, consistent with good medical practice. The composition is administered and dosed in accordance with good medical practice taking into account the clinical condition of the individual patient, the site and method of administration, scheduling of administration, and other factors known to medical practitioners. The "effective amount" for purposes herein is thus determined by such considerations as are known in the art.

### Human Gene Therapy

The coding sequence for the HPV16 specific ribozyme is cloned into a vector as described herein and used in human gene therapy (Mulligan, 1993, Science 260:926-932). In one embodiment, a U6 promoter was cloned into the vector and positioned immediately before the ribozyme coding region. The U6 promoter is a eukaryotic pol III promoter capable of driving transcription of the ribozyme using the host cell's RNA polymerase II (Das, 1988, EMBO J. 7(2):503.512). The use of a retroviral vector for carrying the encoded ribozyme aids in the integration of the ribozyme coding sequence within the cell's genomic DNA, thus providing long-term production of the anti-HPV16 ribozyme within the cell (Chatterjee and Wong, 1993, Methods: Companion to Methods in Enzymology 5(1):51-59).

To deliver the ribozyme-encoding vector to the target cells, a Lipofectin-based liposomal delivery system is used. The use of liposomes aids in getting the vector-ribozyme DNA to the cell without being degraded since the liposome acts as a protective barrier from nucleases (Sullivan, 1993, Methods: Companion to Methods in Enzymology, 5(1):61-66). The cells take in the vector-containing liposomes via the naturally occurring process of endocytosis. The advantage of using the Lipofectin reagent is that it allows the liposome; once taken into the cell, to bypass degradation by lysosomal enzymes which is the usual fate of endocytic material (Felgner, et al., 1993, Methods: Companion to Methods in Enzymology 5(1):67-75). In a preferred embodiment, rihozymes directed against either or both of E6 and E7 are administered in combination with immunological agents such as LAK cells or chemotherapeutic agents such as cisplatin, which has use in cervical cancer. Delivery of a ribozyme to the cervical area can be by either painting or injection.

In addition to the vector described above, other vectors for the delivery of HPV-specific ribozymes can be used in gene therapy. Such vectors are described in more detail below.

The methods used with and the utility of the present invention can be further shown by the following examples.

### Materials and Methods

**Enzymes and chemicals**. All restriction enzymes used were from either Bethesda Research Laboratories (BRL) or Boehringer Mannheim Biochemicals. The buffers for restriction enzymes were supplied by the manufacturer. T4 DNA ligase and the sequencing kit were obtained from Pharmacia. The *in vitro* transcription kit and relevant enzymes were obtained from Promega. Bovine calf serum, antibiotics (penicillin and streptomycin), L-glutamine, sodium pyruvate, phosphate-buffered saline (PBS) and Dulbecco modified Eagle medium (DMEM) were purchased from GIBCO.

T7 RNA Polymerase used was manufactured by US Biochemicals (USB). With the exception of T7 RNA Polymerase, the buffers for enzymes used were supplied by the manufacturer. The T7 RNA Polymerase transcription buffer consisted of the following: 40 mM Tris pH 8.0, 6 mM MgCl₂, 5 mM DTT, 1 mM Spermidine, 1% Triton-X 100. Synthetic DNA templates used for *in vitro* transcriptions and cloning were produced using an Applied Biosystems 392 DNA synthesizer.

**Recombinant DNA techniques.** Unless stated otherwise, the techniques were performed as described in Sambrook, et al. (1989, Molecular Cloning: A Laboratory Manual (2d ed.), Sections 1.25-1.28, 1.60-1.61, 1.68-1.69, 1.82-1.84, 6.9-6.13, 6.46-6.48) the entire disclosure of which is incorporated herein by reference.

**Cleavage of HPV substrates.** Cleavage was carried out in 12 mM MgCl₂, 2 mM spermidine and 40 mM Tris, pH 7.5 using methods previously published (Hampel and Tritz, 1989, Biochem. 28:4929-4933). All reactions were carried out at 37°C, with 25 nM ribozyme and 50 nM substrate for 60 minutes unless otherwise indicated. The reference reaction was native (-)sTRSV sequence S17/R53 at 10 nM and 100 nM for the times shown (Hampel and Tritz, 1989, Biochem. 28:4929-4933).

**P**^{**32**} **labelling.** Substrate and ribozymes were labelled with a P³²-CTP by transcription from synthetic DNA templates using T7 RNA polymerase as previously described (Hampel and Tritz, 1989, Biochem. 28:4929-4933) and reaction products separated on 15-18% polyacrylamide gels in 7M urea.

**Construction of the ribozyme.** The ribozyme was constructed by T7 transcription from complementary synthetic DNA templates. This was carried out as previously described (Hampel and Tritz, 1989, Biochem. 28:4929-4933).

**Construction of Plasmids and Vectors Containing RHPV.** Coding and non-coding strands for RHPV were synthesized and HPLC purified. The strands included in EcoRI site, the ribozyme coding region, a poly·T termination signal for RNA Polymerase III, and a BamHI site. The two strands were then annealed by adding an equimolar amount of each and incubating in H₂O at 90°C for 5 minutes, then allowed to slowly cool down to room temperature over a 30-minute period. The resulting double-stranded fragment was digested with Eco RI and BamHI. The digestion products were run on an agarose gel, and the ribozyme coding fragment was isolated and purified.

The plasmid pHC (Altschuler, 1992, Gene, 122:85-90) was digested with EcoRI and BamHI, and the fragment was isolated and purified as above. The RHPV434 or RHVP419 fragment was then ligated into pHC, and the ligation mixture was used to transform competent DH5α bacterial cells. Single colonies were selected and grown in CircleGrow bacterial media, and plasmids extracted and purified using a standard miniprep protocol (Sambrook et al., 1989, Molecular Cloning: A Laboratory Manual (2d ed.), Sections 1.25-1.28, 1.60-1.61, 1.68-1.69, 1.82-1.84, 6.9-6.13, 6.46-6.48). The plasmids were screened for incorporation of the RHPV434 or RHVP419 insert. A colony that incorporated the insert was then sequenced using the Sequenase Version 2.0 enzymes and protocol to verify the proper DNA sequence. The resulting plasmid was termed pHC-434 or pHC-419 respectively.

The ribozymes are cloned into a Moloney retrovirus-based expression vector for *in vivo* testing in human cells transformed with HVP-16. The cloning scheme is as follows. The ribozyme oligos are synthesized with a Pol III termination signal and EcoR1/BamH1 termini. These are then cloned into pHC (Altschuler, 1992, Gene, 122:85-90), the standard bacterial expression vector used in a preferred embodiment. The ribozyme is cut out with EcoR1 HindIII and cloned into pU6 which is a Bluescript vector containing a mouse U6 promoter (Das, 1988, EMBO J. 7(2):503-512). The insert containing the U6 promoter is then cloned into the BamH1 site of pZIP-NeoSV(X) (Cepko, et al., 1984, Cell 37:1053-1062).

pHC-434 and pMU6, a plasmid which contains an RNA polymerase III promoter region (Das, 1988, EMBO J. 7(2):503-512) were digested with Eco RI and Hind III. The RHPV434 fragment, which retained the hairpin cassette region, and the pMU6 fragment were isolated and purified as described above. Ligation and bacterial transformation of the two fragments was carried out as described above. Colonies were screened and sequenced as described above. The resulting plasmid was termed pMU6-434.

**Screening of HPV sequence (SEQ ID No:1) for cleavage site**. HPV16 sequence data was obtained through Gen Bank. HPV16 E6 and E7 regions were inspected for potential target sequences as described above. All potential sites containing potential target sequences were tested, and ribozymes that showed significant catalytic activity were further developed. RHPV434 and RHVP419 are examples of ribozymes that showed significant catalytic activity.

The general principles of the present invention can be more fully appreciated by reference to the following non-limiting examples.

### Example 1

In the preferred embodiment of the present invention as shown in Figure 2, Loop 3 and Helix 4 are constructed to include the stable RNA hairpin sequence.

5'-GGAC UUCG GUCC -3' (SEQ ID No:5) resulting in the "tetraloop" modification (Cheong, et al., 1990, Nature, 346:680-682; Varani, et al., 1991, Biochem. 30:3280.89). As a result Helix 4 is extended by four base pairs over the non-modified sequence. Further, the GUU sequence of Loop 3 is replaced with the sequence UUCG.

To determine the activity of the ribozyme, it is added to a substrate RNA at a ratio of 1:30 and the time course of cleavage studied as parameters are varied. The reaction is carried out in 12 mM MgCl₂, 40 mM Tris, pH 7.5 and 2 mM spermidine over 150 minutes. For temperature dependence, the rate of cleavage of a ribozyme containing the tetraloop modification is tested over a temperature range and compared to the control reaction at 37°C. The reaction products are analyzed on polyacrylamide/urea gels. The bands are cut out and counted in a liquid scintillation counter. In the control reaction only 2% of the substrate remains after 150 min. indicating that the ribozyme must interact with multiple substrates during the course of the reaction since there were 30 times as much substrate as ribozyme. Further, the amount of the ribozyme remains the same and unaltered as expected of a catalyst.

In the temperature dependent study of the tetraloop modification compared to the prior art, the activity of the ribozyme was measured at 20°C, 27°C, 33°C, 37°C, 41°C and 45°C.

The reaction showed a temperature dependence similar to that which would be expected of a reaction involving base paired RNA molecules. The Arrhenius plot of the data gives a temperature optimum of 37°C for the reaction. Higher temperatures reduce the reaction rate with a very rapid rate reduction about 41 °C consistent with a melting out of the catalytic RNA structure. At 50°C, no reaction was detectable. The reaction rate at temperatures below 37°C showed a linear reciprocal temperature dependence consistent with a classical lowering of the energy of the activation for the reaction. The slope of the line in the Arrhenius plot gave an energy of activation of 19 Kcal/mole which is close to that found for catalysts fitting the hammerhead cleavage mechanism (13.1 Kcal/mole) (Uhlenbeck, 1987, Nature 328:596-600).

The example shows that a ribozyme with the tetraloop modification is more active and more thermally stable than the prior art. This form of the ribozyme remains active at 45°C while the nonmodified ribozyme lost most of its activity at this temperature.

It was concluded from this experiment that Loop 3 does not have a conserved or invariant base sequence and that Helix 4 can be extended into Loop 3 by at least four base pairs with no loss of activity. The four additional base pairs in Helix 4 provide helix stabilization of this region. The secondary folding energy of Helix 4 and Loop 3 in the prior art structure is +0.6 Kcal/mole, while that of the ribozyme having the extended Helix 4 and Loop 3 of the sequence UUCG (tetraloop) of the present invention was determined to be -11.1 Kcal/mole. Thus the presence of the tetraloop sequence increases the folding energy by 11.7 Kcal/mole.

### Example 2

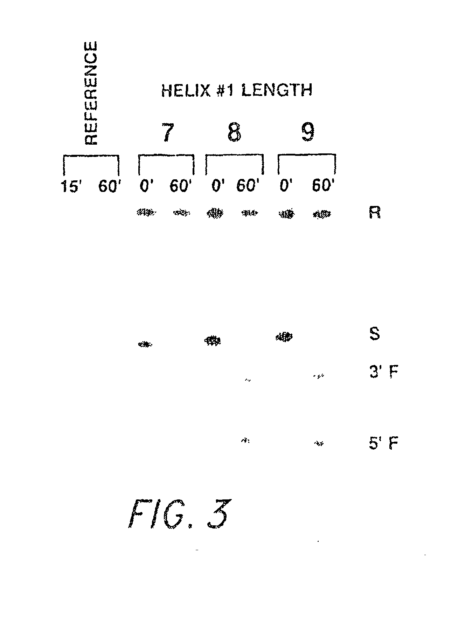

**The cleavage reaction and optimization of helix 1 length for RHVP434.** A cleavage study was undertaken to optimize the length of Helix 1. Figure 3 shows bands on a denaturing polyacrylamide gel identifying the ribozyme, substrate and cleavage products. Three substrates were cleaved by the ribozyme, each with a different length helix 1. The substrates were as follows:

| **Substrate** | **Helix 1 Length** | **% Cleaved** |
|---|---|---|
| 430-ACUG U*GUC CUGAAGA-444 (SEQ ID No:2) | 7 | 5.4 |
| 430-ACUG U*GUC CUGAAGAA-445 (SEQ ID No:3) | 8 | 6.7 |
| 430-ACUG U*GUC CUGAAGAAA-446 (SEQ ID No:5) | 9 | 6.5 |

The most efficiently cleaved substrate was that which had an 8bp helix 1 (SEQ ID No:3) and was used for all further studies. It is referred to as SHPV and the corresponding ribozyme is referred to as RHPV-434 (Figure 2).

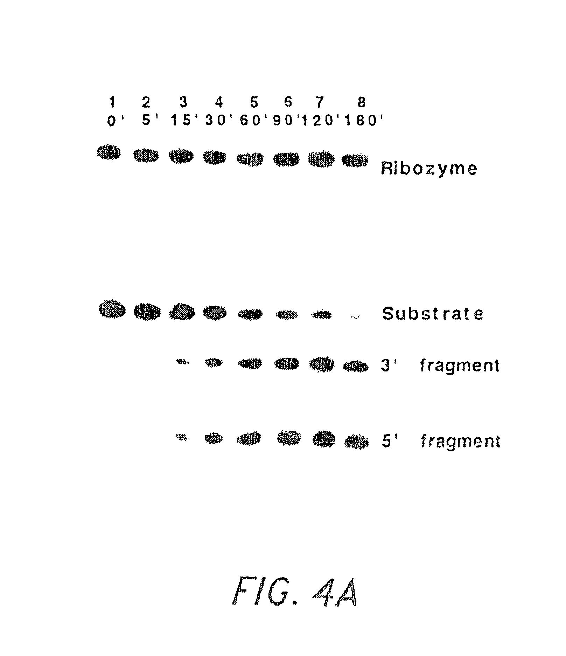

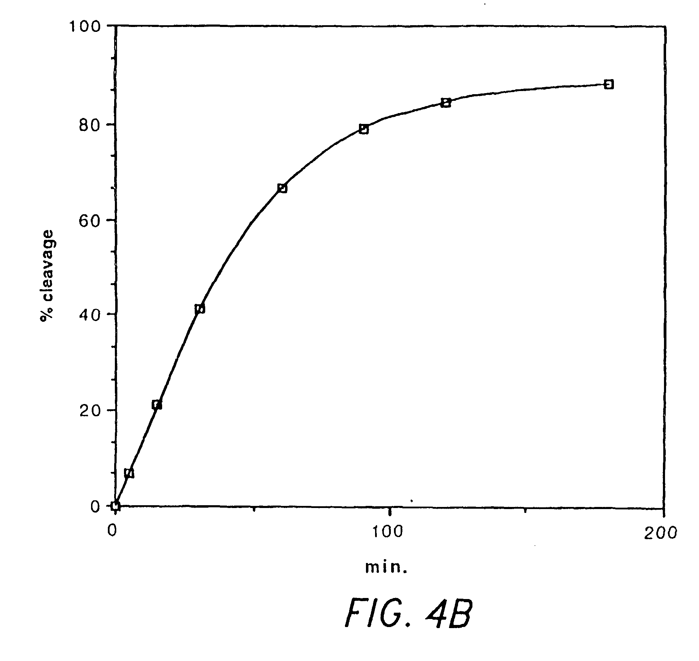

**Time course of cleavage.** The time course for cleavage of SHPV by RHPV.434 was done over a 180 min period (Figure 4). The ribozyme efficiently cleaved the substrate to 88% completion.

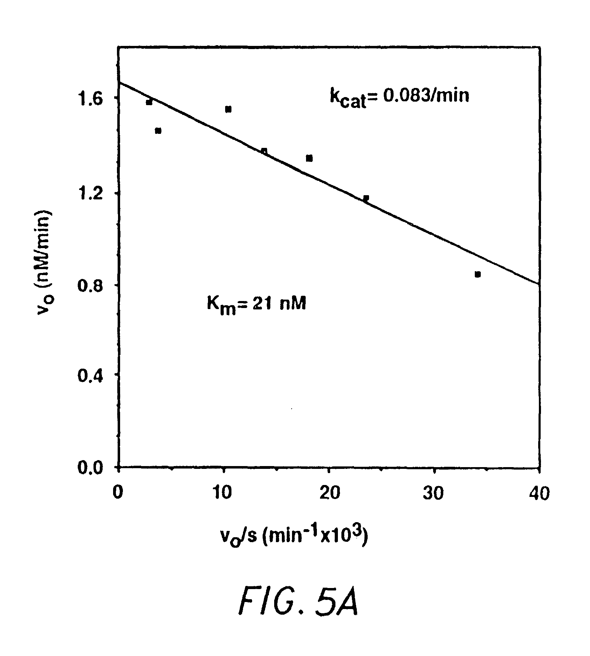

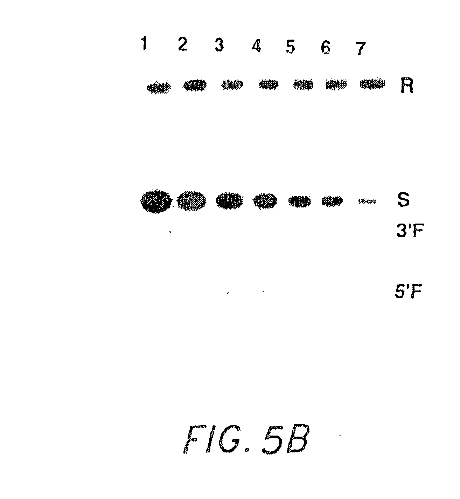

**Kinetic parameters of cleavage.** A Michaelis kinetic analysis of the reaction was carried out by using limiting ribozyme and excess substrate for constant ribozyme concentration and varying substrate concentrations to measure initial velocities (Figure 5). The Kₘ for the reaction was 21 nM and k_{cat} or turnover number was 0.083 min⁻¹. This gives an overall catalytic efficiency (kcat/Km) of 4µM⁻¹ min⁻¹ which is about 7% that of the original native hairpin sequence (Hampel and Tritz, 1989, Biochem. 28:4929-4933).

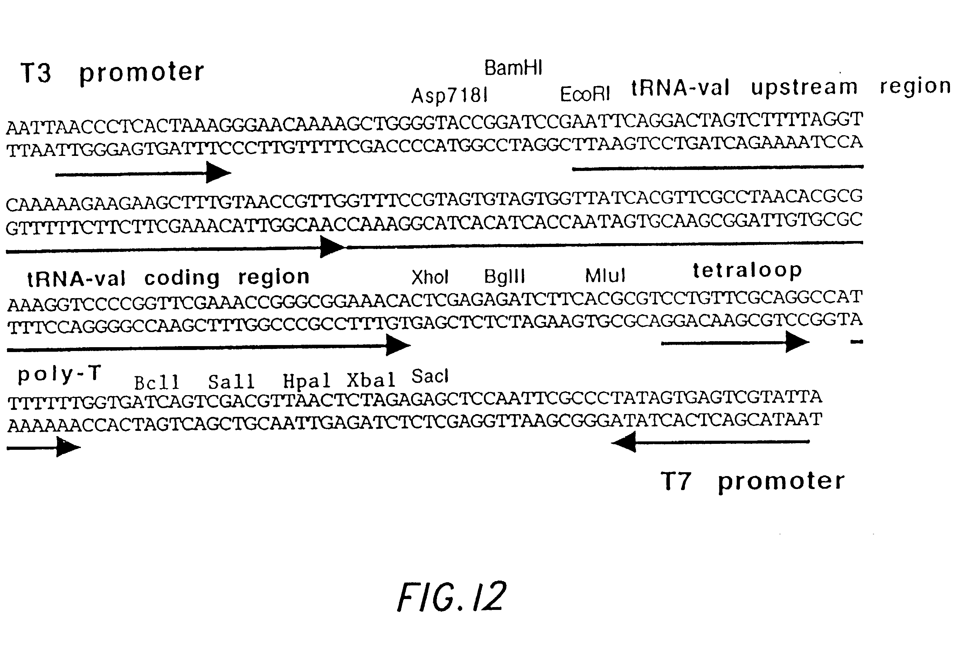

Construction of the vector ptV1 containing a delivery cassette. The vector ptV1 (Figs. 7 and 8) was prepared by cloning a delivery cassette consisting of a new polylinker, a human tRNA^{val} pollll promoter with a human upstream region (Arnold, et al., 1986, Gene 44:287-297), a tetraloop sequence variant, and a poly-T sequence into the vector pBluescript KS (from Stratagene). The human upstream region is shown, e.g., in Figure 12 (see also Arnold and Gross, 1987, Gene 51:237-246).

The steps for constructing the vector are as follows. Both strands of a new 119 nt linker region (Fig. 6) was prepared by chemical oligonucleotide synthesis. This linker region contained various restriction sites, the tetraloop sequence described above, and a poly-T tract to terminate pollll transcription. This was cloned into the Asp718(Kpn1)/Sac1 sites of pBluescript KS (Stratagene) to give pBKSLNKR (not shown). The plasmid pHTV1 (see Amold, et al., 1986, Gene 44:287-297) contains the human tRNA^{val} promoter. The tRNA^{val} gene plus an additional 50 nt of upstream sequence was amplified by PCR with EcoR1/Xhol termini. This was cloned into the corresponding sides of the linker region of pBKSLNKR to give the final ptV1 construct (Figs. 7 and 8).

The ribozyme coding sequences can be cloned into the Xho1/M1u1 sites of ptV1. All chemical syntheses of DNA oligonucleotides were carried out using standard methods on a ABI 392 DNA/RNA synthesizer (using the manufacturer's recommendations for procedures). All constructions of plasmids were done using standard procedures (Sambrook et al., 1989, Molecular Cloning: A Laboratory Manual (2d ed.)).

The tetraloop variant at the 3' terminus of the ribozyme has the sequence CCUG(UUCG)CAGG (SEQ ID NO:16). This sequence is a variant of the highly stable tetraloop sequence GGAC(UUCG)GUCC (SEQ ID NO:5) found in various native RNA sequences (Cheong, et al., 1990, Nature 346:680). In contrast to the native tetraloop, the variant reverses the two sequences forming the helix relative to the original. This difference, relative to the native tetraloop and other synthetic hairpin ribozyme constructs (Anderson, et al., 1994, Nuc. Acids Res. 22:1096-1100), decreases the probability of recombination *in vivo* between the construct and native or other synthetic sequences. This vector construct also has the advantage that the tetraloop variant can be removed and replaced by another tetraloop sequence by simply digesting the vector with Mlu1/ Bstx1 and inserting another tetraloop sequence with appropriate ends.

The ptV1 plasmid has additional advantages based on its components and their arrangement. The pollll promoter gives very high *in vivo* transcription of ribozyme (Yamada et aL, 1994, Gene Therapy 1:38-45). The upstream region improves *in vivo* transcriptional levels of ribozyma (Arnold and Gross, 1987, Gene 51:237-246). The tetraloop variant gives improved stability to the ribozyme by protecting the 3' terminus from degradation by a 3' exonuclease. The poly-T sequence allows for efficient termination of transcription originating at the pollll promoter. Restriction sites (XhoI/MluI or BGIII/MluI) after the tRNA coding region are suitable for inserting ribozyme coding sequences. For example, a BgIII overhang can be ligated to a BamH1 overhang. Transcription initiates at the tRNA coding region and terminates efficiently after the poly-T tract is transcribed into a poly-U portion of the ribozyme.

The whole delivery cassette can be easily digested from ptV1 as a unit and. cloned into the corresponding restriction sites of viral expression vectors including Moloney-retrovirus based vectors, adenovirus, adeno-associated virus, and the like. This approach has been used to construct a series of constructs for delivery of HPV-specific ribozymes to living cells.

Construction of the vactor for delivery of human papillomavirus-specific ribozymes to human cells. Our constructs containing the delivery cassette, a hairpin ribozyme sequence and a retroviral vector have been designed to specifically cleave HPV sequences. The ribozyme, RHPV434, was designed to cleave site 434 in the sequence of human papillomavirus HPV-16 (GenBank accession #K02718). The 434 site is in the E6/E7 sequence of human papillomavirus (HPV) which has been found in human carcinoma (DiPaolo, et aL, 1993, Critical Reviews in Oncogenesis 4:337-360). The retroviral vector used was pZIP. NeoSV(X)1 (Cepko, et al., 1984, Cell 37:1053-1062) which is a Moloney retrovirus-based vector used in human genetic engineering (see, for example, the 1994 RAC Application of Wong-Stall).

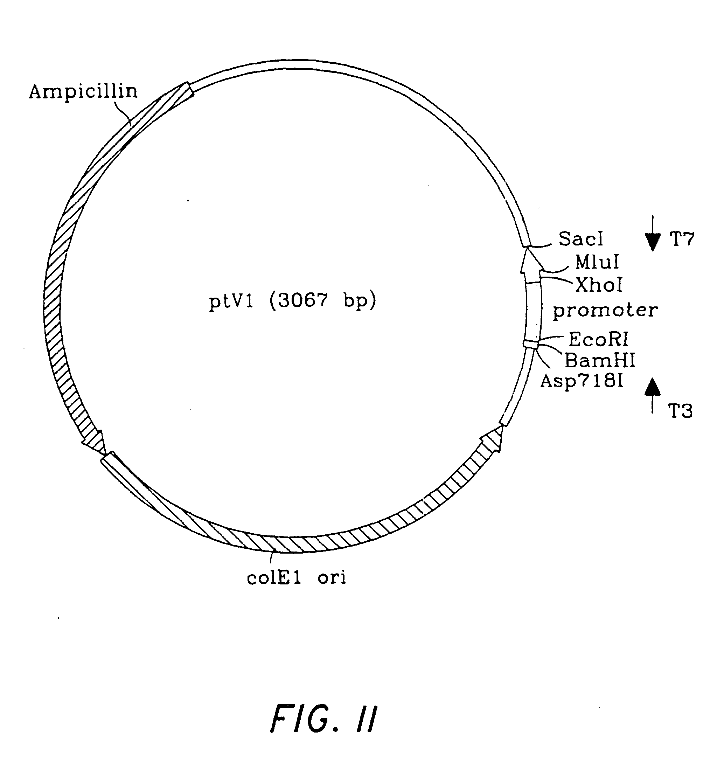

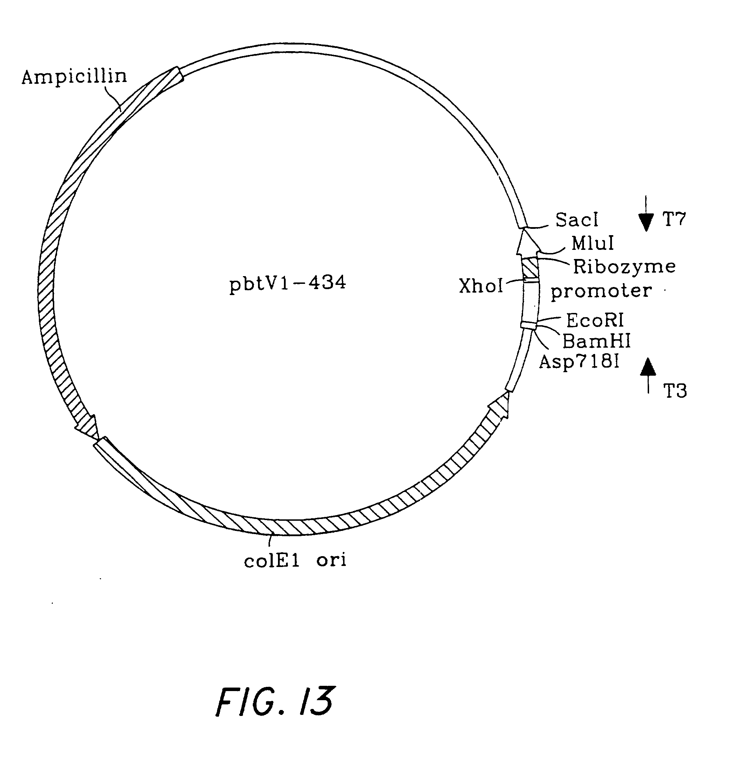

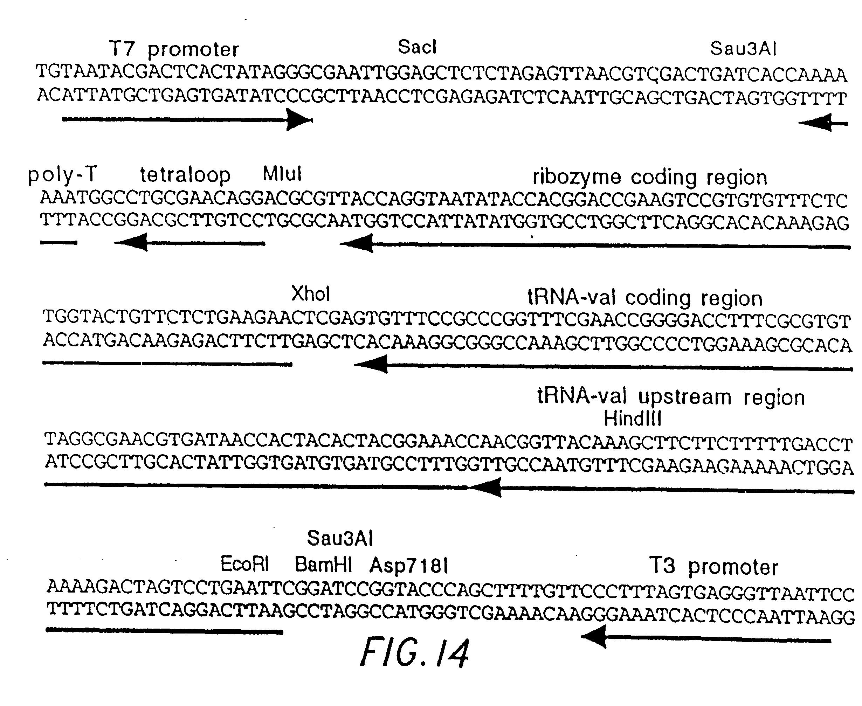

The DNA corresponding to an HPV-16 specific hairpin ribozyme for site 434 in the HPV-16 sequence was synthesized and cloned into the Xho1/M1u1 sites of vector ptV1 as described above to give the plasmid pBtV1-434 (Figs. 9 and 10). The vector pBtV1-434 was digested with Sau3A1 and a 226 bp insert containing the delivery cassette with its inserted ribozyme was isolated following separation by agarose gel electrophoresis using standard methods. Digestion of this plasmid with Sau3A1A produces a number of fragments. The 226 bp fragment (cut between the BamH1 and Bell sites) was identified by running a control digest of pBluescript KS side-by-side with the digest of pBtV1-434; the 226 bp fragment was seen only in the pBtV1-434 digest. This 226 bp fragment was cloned into the BamH1 site of pZIP-Neo SV(X)1 in both orientations to give the plasmids pZIPV1-434(syn) (Figs. 11 and 12) and pZIPV1-434(anti) (Figs. 13 and 14), respectively. The "syn" orientation refers to constructs in which the inserted pollll promoter is in the same orientation as the pollll promoter of the retrovirus. The "anti" constructs have the inserted pollll promoter is in the opposite orientation relative to that of the pollll promoter of the retrovirus.

In these constructs, the RHPV434 ribozyme coding region is downstream and therefore transcribed from a very powerful pollll promoter in a Moloney retrovirus-based vector. This pollll promoter gives very high transcription of *in vivo* effective ribozyme (Yamada, et al, 1994, Gene Therapy 1:38-45). The upstream region improves *in vivo* transcriptional levels of ribozyme (Arnold and Gross, 1987, Gene 51:237-246). The tetraloop gives improved stability by protecting the 3' terminus of the ribozyme against nuclease degradation. The poly-T sequence terminates transcription from the pollll premoter.

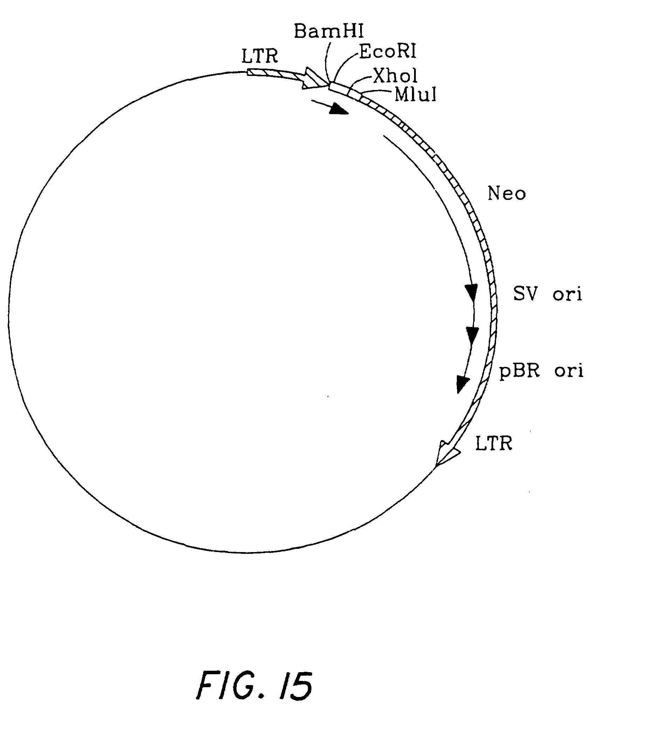

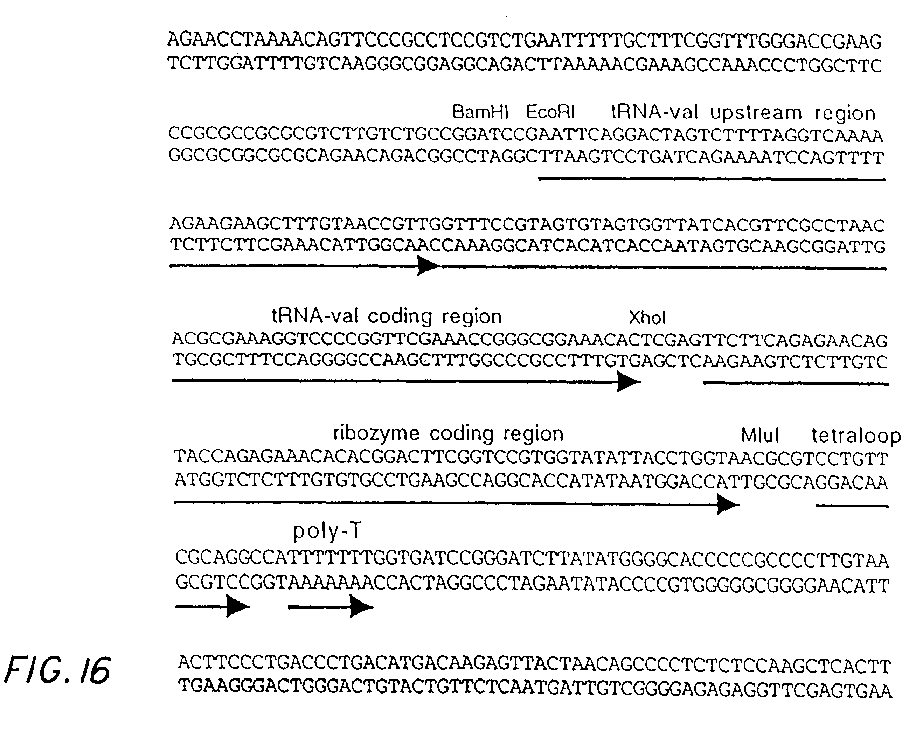

A corresponding inactive construct was also made. The inactive construct had a mutation changing an AAA to a CGU in loop 2 of the hairpin ribozyme cloned into ptV1 (as described above). The catalytically inactive ribozyme is encoded by the pBtV1-434(i) plasmid (Figs. 15 and 16).

To verify that these and similar constructs have activity *in vivo,* constructs were transfected into tissue cultured calls and production of ribozyme was assayed.

### Example 3

***In vivo* testing.** *In vivo* tasting was performed using the HFV-16 specific constructs. The pZIPv1-434(syn) and pZIPV1-434(anti) vectors were stably transfected into human CXT1 cells using standard methods (Sambrook, et al., 1989, Molecular Cloning: A Laboratory Manual, 2d ed.). The CXT1 cell line was derived from a spontaneous human cervical cancer tumor and has been shown to express the E6 and E7 proteins of HPV16.

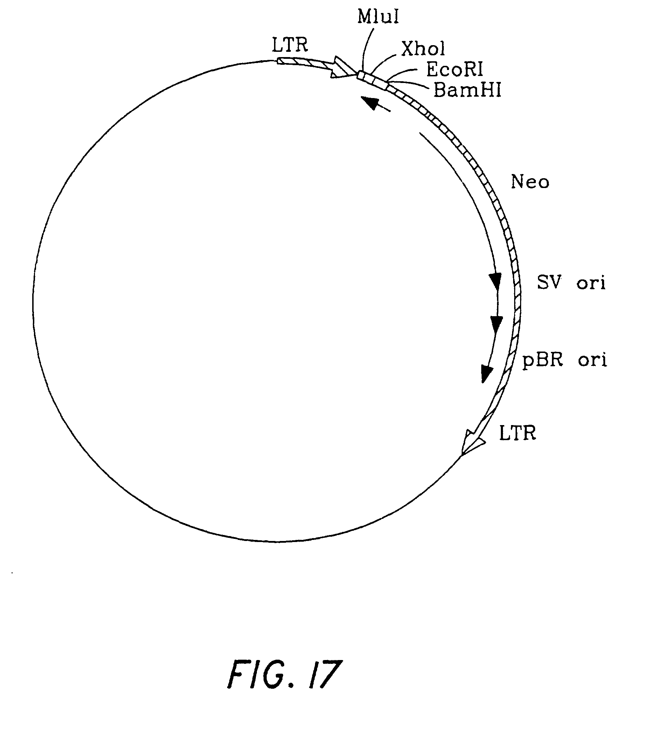

**The ribozyme is expressed in vivo by the delivery eassette in the retroviral vector.** Ribozyme expression was determined by RNase protection assays (Fig. 17; using the method of Lee and Costlow, 1987, Methods Enzymol. 152:633-648). The CXT1 cells were transfected with the pZIP-Neo-SV(X), pZIP·V1434(anti), and pZIP·V1434(syn) constructs and transformants were selected as resistant to the drug G418: RNA was isolated from the transfected cells using the acid phenol method (Sambrook et aL, 1989, Molecular Cloning. A Laboratory Manual, 2d ed.). The probe used was transcribed with T7 RNA polymerase from the plasmid pBtV1-434 (Figs. 13 and 14) which was previously cut with BamH1; the RNA transcript of 265 nt thus produced was used as the probe. RNase protection was carried out and products separated by gel electrophoresis using standard procedures (Sambrook at aL, 1989, Molecular Cloning: A Laboratory Manual, 2d ed.).

Referring to Fig. 17, the arrow indicates the 160 nt size of the protected ribozyme transcript from the pollll promoter. Ribozyme is seen in the pZIPV1-434(syn) lane whereas none is seen in the lanes corresponding to RNA from cells transfected with pZIP-Neo-SV(X) and pZIP-V1434(anti). Similarly, no 160 nt fragment is seen in the control lane for cells that were not transfected ("NT" lane). The other lanes are controls showing that the ³²P probe is intact ("probe" lane) and that RNase is active for digestion of cellular RNA and probe ("RNase +" lane).

Note that only a 160 nt band was seen. If a longer 230 nt band had been seen, it would have indicated transcription originating from the pollll promoter of the vector. The presence of only the 160 nt fragment shows that the ribozyme transcript was transcribed from the pollll promoter of the delivery cassette, and not from the vector promoter.

**The hairpin ribozyme appeared to lower expression of the E6 mRNA in vivo.** To determine if ribozyme expression affects HPV expression, an assay based on reverse transcription (RT) and amplification by the polymerase chain reaction (PCR) was used. The CXT1 cells stably transfected with the pZIP·Neo·SV(X), pZIP·V1434(anti), and pZIP·V1434(syn) constructs and selected by 6418·resistance were used as a source of HPV mRNA. The mRNA was isolated by the acid Phenol method and further purified using binding to paly-A using standard methods (Sambrook et al., 1989, Molecular Cloning: A Laboratory Manual, 2d ed.). The mRNA was reverse transcribed to make cDNA which was PCR amplified with E6 specific primers to detect a loss of E6 mRNA. The primer used for reverse transcription (called "16E7R"), complementary to the 3' end of E6 mRNA was: 5' TTATGGTTTCTGAG 3' (SEQ ID NO:12).

One of the PCR primers was an anchored probe specific to E6 cDNA which amplifies the lower strand of the E6 cDNA whether or not it is cleaved by the ribozyme. The other two PCR primers were specific for amplification of the upper strand of E6 cDNA. One of these upper strand primers is 5' of the ribozyme cut site at nt 434 and the other is 3' of the ribozyme cut site. These three primers were: the anchored primer, called E7X, having the sequence 5' CCCTCTAGAGGCACACAATTCCTAGTG 3' (SEQ ID NO:13); the primer 3' of the ribozyme cut site, called E6-U2, having the sequence 5' CACGTAGAGAAACCCAGC 3'(SEQ ID NO:14); and the primer 5' of the ribozyme cut site, called E6-16U, having the sequence 5' CAGCAATACAACAAACCG 3' (SEQ ID NO:15).

PCR amplification was performed using standard conditions: 25 cycles at 94°C for one min for melting, then 65°C for 45 sec for annealing and 72°C for one min for polymerization using an automated thermocycler (Perkin Elmer model 480). Five units of Amplitaq polymerase (Perkin Elmer), 100 µM of each NTP, 10 ng of primer E6-16U (SEQ ID NO:15), 10 ng of primer E6-U2 (SEQ ID NO:14) and 20 ng of primer E7X (SEQ ID NO:13) were used.

This combination of three primers gave two PCR products of approximately 300 nt and 500 nt respectively in cells in which the E6 mRNA was intact. However, in cells in which the ribozyme cut the E6 mRNA, the smaller of the two PCR products of 300 nt was more predominant because of the molar ratio of ribozyme-cut E6 mRNA to complete-length E6 mRNA. The products were separated by gel electrophoresis.

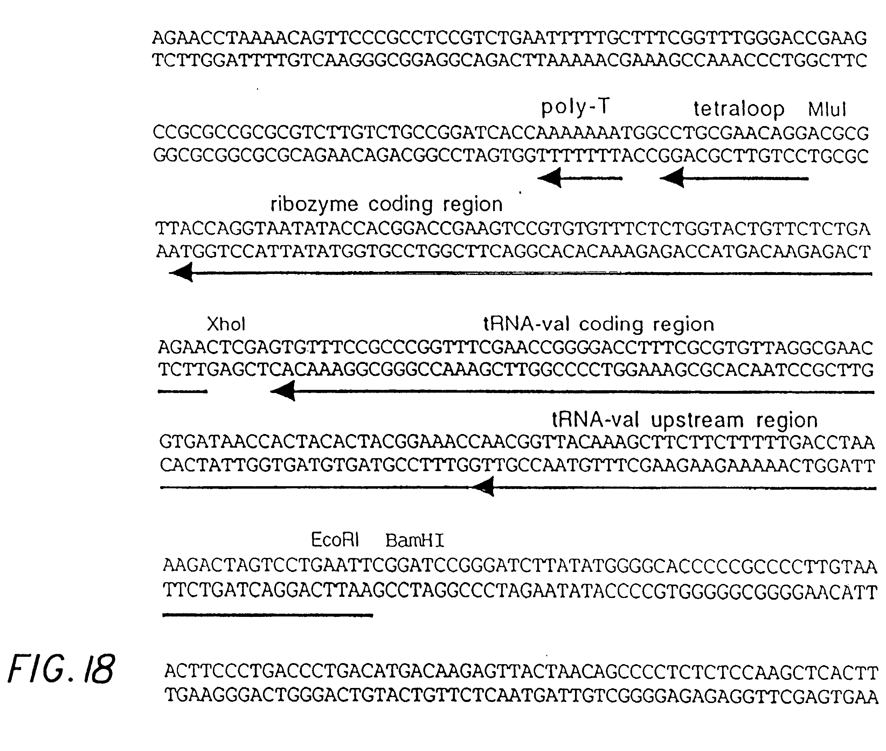

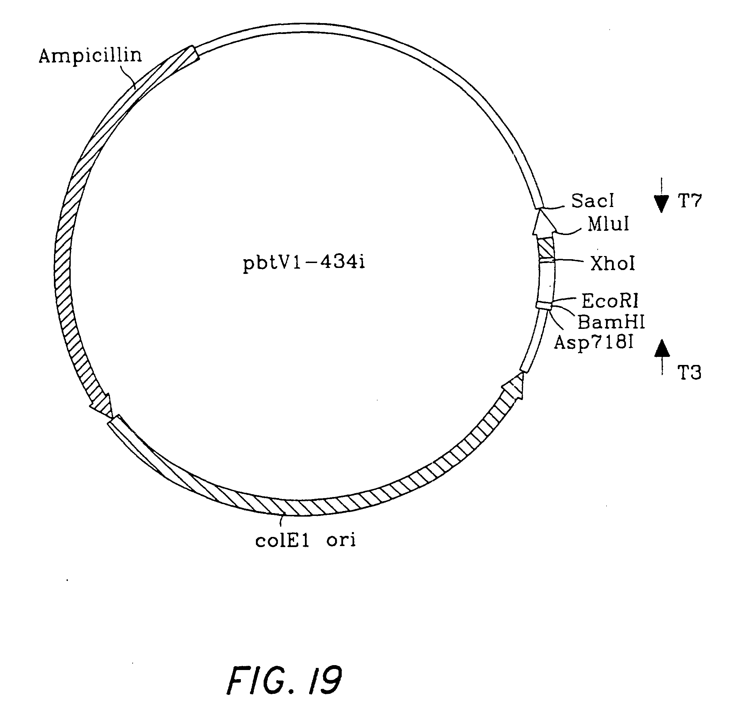

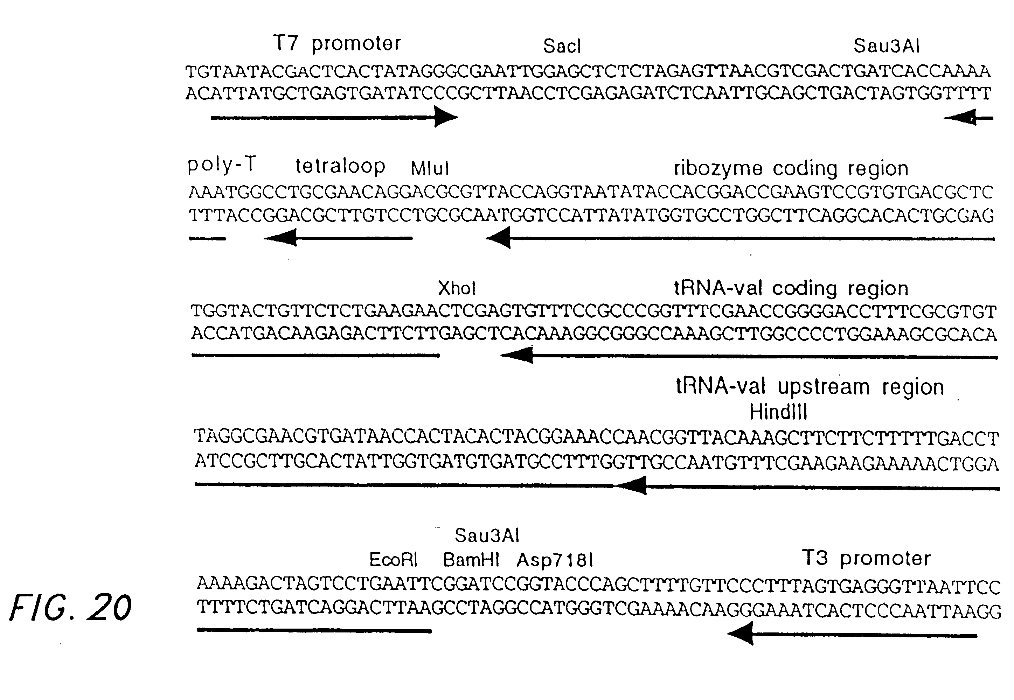

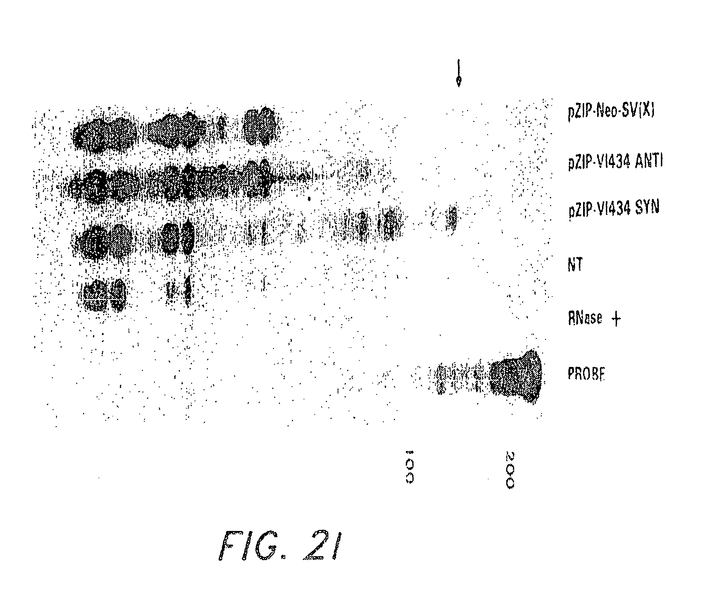

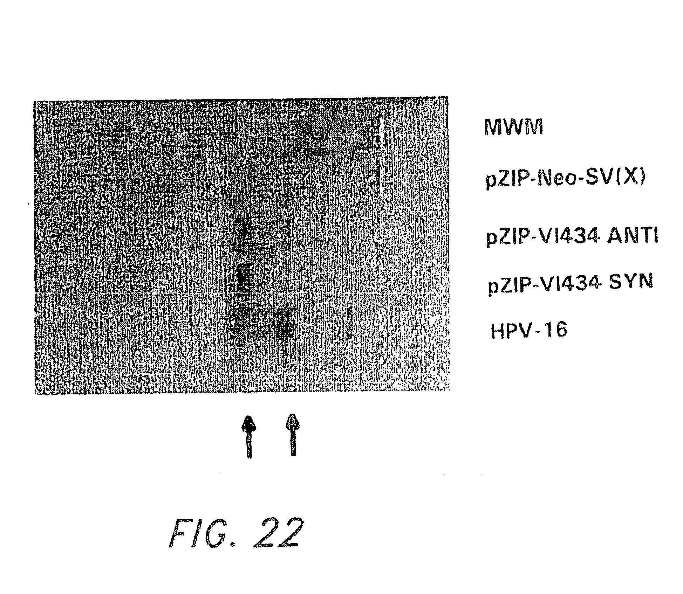

Fig. 18 shows that the RT and PCR assay detects the presence of HPV E6 mRNA in infected cells. This figure also shows that the ribozyme cleaves the E6 mRNA in transfected cells that express the ribozyme. The arrows indicate the positions where the two PCR amplified products migrate on a gel, with the leftmost arrow indicating the position of the 300 hp fragment and the rightmost arrow indicating the position of the 500 bp fragment as determined from the positions on the gel of the molecular weight markers ("MWM" lane). The positive control lane ("HPV-16") showed bands at both positions indicating that E6 mRNA is present in the CXT1 cells and that the assay successfully amplified both fragments. Cells transfected with the pZIP-Neo-SV(X) plasmid also serve as a control because the plasmid does not encode an HPV-specific ribazyme, and bands were seen at both positions.

Amplification using the three primers can be used to identify the presence of HPV-16 in human tissue. A sample containing RNA from human tissue can be subjected to the ribozyme and subsequently amplified using the three primers. The ribozyme will only cut RNA having the HPV-16 binding sequence. Thus, samples containing HPV-16 RNA will be cut by the ribozyme and result in preferential amplification of the shorter product produced by the primer 5' of the ribozyme cut site.

### Example 4

Selection of a non-HPV-16 transformed cell line with HPV-16 specific ribozymes and trartsfection with an HPV-16/E7 expression vector. HeLa cells are stably transfected with constructs capable of expressing an HPV ribozyme specific for site 434 in HPV. These constructs express the ribozyme using a pollll promoter of either mouse U6 (MU6) or human tRNA^{val} (tV1) origin. Verification of ribozyme expression is determined by using RNA probes and RNase protection assays.

Ribozyme specific for site 434 of HPV is cloned into vectors with the ribozyme construct transcribed from either the mouse U6 (MU6) or human tRHA^{val} (tV1) pollll promoter. Both bacterial plasmid constructs and Moloney retrovirus based constructs are made. The bacterial plasmid constructs have the human tRNA^{val} promoter cloned into the pBluescript(KS) plasmid (available from Stratagene). The 434 specific ribozyme is cloned into pBluescripts with the tRNA^{val} promoter at the multiple cloning site to give the plasmid pBTV1-434. The insert is removed from this plasmid by cutting with restriction enzyme Sau3A1 and the appropriate fragment size containing the insert is isolated using standard gel purification methods. The insert is then subcloned into the BamH1 site of pZIP-neo which is a Moloney marine leukemia virus based plasmid, to give the plasmid pZIP-434 with the insert in either the "syn" or "anti" configuration.

HeLa cells are transfected with the ribozyme constructs and stable transfectants are selected using the G418-resistance selection. Cells are grown up and RNA is isolated from the cells for use in an RNase protection assay to identify expressed ribozyme. Tha probe to be used for identification of the rihozyme is from a plasmid with the ribozyme inserted between a T3 and a T7 promoter. These two plasmids are called pMU6-434A for the U6 promoter-ribozyme construct and pBTV1-434 for the tRNA^{val} promoter-rbozyme construct. The probe for the MU6 promoter construct is produced by linearizing the MU6-containing plasmid, pMU6-434A, with EcoRI and transcribing the linearized plasmid with T3 RNA polymerase using standard procedures. This probe, which is 110 nt long, anneals to ribozymes produced from both the long terminal repeat (LTR) of the pZIP-434(syn) plasmid and the pollll promoter of plasmids pZIP-434(syn) and pZIP-434(anti). No ribozyme can be produced by the LTR from the pZIP-434(anti) configuration. After annealing and RNase digestion, the protected fragment produced from the LTR is 77 nt in length, whereas the protected fragment produced from the pollll. promoter is 64 nt long.

The probe used with the tV1 promoter construct is produced by linearizing the plasmid pBtV1-434 with BamHI and transcribing the plasmid DNA with T7 RNA polymerase using standard polymerization conditions. This probe, which is 265 nt long, anneals to ribozymes produced by both the LTR of pZIPV1-434(syn) and the pollll promoter of plasmids pZIPV1-434(syn) and pZIPV1-434(anti), but the sizes of the protected fragments differ. After RNase digestion, the protected fragment from the LTR is 230 nt in length, whereas the protected fragment produced from the pollll promoter is 160 nt long.

Cells are infected with a retroviral vector capable of expressing E6/E7 genes. Comparative levels of the three mRNA species for these two proteins (called E6E7, E6(I)E7 and E6(II)E7; the first two being the major species and the third being the minor species) are measured using a reverse transcriptase and polymerase chain reaction (RT/PCR) assay as outlined in Comelissen et al. (*J*. *Gen. Virol.* 71:1243-1246, 1990.

The primers used in the RT/PCR assay are as follows. Primer 1 is 5' NNNAAGCTTCTGCAATGTTTCAGGACCC 3' (SEQ ID NO:17) and primer 2 is 5' NNNGGATCCCCATTGGTACCTGCAGGATC 3' (SEQ ID NO:18). Primer 2 is the primer used in the RT reaction; both primers 1 and 2 are used in the PCR reaction. The RT/PCR reaction yields fragments of the following sizes: 791 bp corresponding to E6E7 product; 608 bp for the E6(I)E7 product and 491 bp for the E6(II)E7 product.

Because ribozyme 434 cleaves the two major species of mRNA (E6E7 and E6(I)E7) and not the minor species (E6(II)E7), the minor species serves as an internal reference standard in the assay. Levels of the two major species (the 791 bp and 608 bp products) relative to the minor species (the 491 bp product) are measured to determine the *in vivo* efficacy of the 434 ribozyme. The major species show a 10-50% reduction relative to the internal minor species product when the ribozyme is expressed. As controls, cells transfected only with the ribozyme vector not containing the ribozyme insert and cells transfected with the inactive ribozyme construct are also used. The RTIPCR assay shows no decrease in the two major species relative to the minor species when these plasmids are transfected.

## Claims

1. A synthetic hairpin ribozyme which binds to and cleaves a target site in a human papilloma virus transcript, said target site selected from the group consisting of target sites represented by: and wherein "*" indicates the cleavage site.

2. A synthetic hairpin ribozyme as in claim 1 which includes a tetraloop consisting of SEQ ID NO: 5.

3. A synthetic ribozyme as in claim 1 consisting of the complete sequence as set forth in SEQ ID NO: 6.

4. A method of constructing a ribozyme to cleave a human papilloma virus transcript comprising the step of constructing a hairpin ribozyme wherein a binding site on the ribozyme includes a sequence noncomplementary to the cleavage site on a human papilloma virus and a binding region complementary to the sequences on either side of the cleavage site where the binding regions are complementary to sequences selected from the group consisting of: and
wherein "*" indicates the cleavage site.

5. A method of constructing a ribozyme to cleave a human papilloma virus transcript according to claim 4 wherein the step of constructing said hairpin ribozyme includes incorporating a tetraloop having the sequence set forth in SEQ ID NO: 5.

6. A vector comprising a DNA sequence coding for said ribozyme according to claim 1, the DNA being operatively linked to expression control sequences.

7. A host cell transformed with a vector according to claim 6 and which is capable of expressing said ribozyme encoded by said vector.

8. An in vitro method for detecting a human papillomavirus-16 (HPV-16) in a human tissue extract comprising:
a. exposing RNA from a sample of human tissue to a ribozyme according to claim 1, which binds to a nucleotide sequence from HPV-16 RNA, such that HPV-16 RNA present in said sample is cleaved by said ribozyme;
b. amplifying the cDNA using primers complementary to: a 5' end of a full-length HPV-16 transcript, a fragment 5' of the ribozyme cleaving site of the full-length HPV-16 transcript, and a fragment 3' of the ribozyme cleavage site of the full-length HPV-16 transcript; and
c. identifying DNA fragments amplified, such that a larger DNA fragment represents a full-length HPV-16 transcript and a smaller sized DNA fragment represents the fragment resulting from ribozyme cleavage of the full-length HPV transcript,
wherein if HPV-16 RNA is present in the sample, a preponderance of the smaller fragment is identified relative to the larger fragment.

9. The method of claim 8, wherein the HPV-16 RNA cleaved by the ribozyme is an E6 transcript of HPV-16.

10. The method of claim 8, wherein the ribozyme comprises the sequence of SEQ ID NO:6.

11. The method of claim 8, wherein the human tissue is cervical tissue.

12. The method of claim 8, additionally comprising the step of producing cDNA from the RNA present in the sample after the exposing step and before the amplifying step.

13. The ribozyme of claim 1, for use in medical treatment.

14. The ribozyme of claim 13, for use in treatment of cervical cancer or a pre-cancerous condition of the cervix.

15. The ribozyme of claim 13, suspended in a lipofectin-based liposomal delivery system.

16. The ribozyme of claim 13, in combination with an immunological agent or a chemotherapeutic agent.

17. The ribozyme of claim 13, in combination with LAK cells.

18. The ribozyme of claim 13, in combination with cisplatin.

## Patentansprüche

1. Synthetisches Hairpin (Haarnadel)-Ribozym, welches sich an eine Zielstelle in einem menschlichen Papillomavirus-Transkript bindet und welches eine solche Zielstelle spaltet, wobei die besagte Zielstelle ausgewählt wird aus der Gruppe, die aus den Zielstellen besteht, welche dargestellt sind durch: und wobei "*" die Spaltstelle angibt.

2. Synthetisches Haarnadel-Ribozym gemäß Anspruch 1, welches eine Tetraschleife umfasst, welche aus der SEQ ID NO: 5 besteht.

3. Synthetisches Ribozym gemäß Anspruch 1, welches aus der vollständigen Sequenz besteht, wie sie in der SEQ ID NO: 6 dargelegt ist.

4. Verfahren für den Aufbau eines Ribozyms zum Spalten eines menschlichen Papillomavirus-Transkripts, welches den Schritt des Aufbaus eines Haarnadel-Ribozyms umfasst, wobei eine Bindungsstelle auf dem Ribozym eine Sequenz enthält, welche nicht komplementär zu der Spaltstelle auf einem menschlichen Papillomavirus ist, und einen Bindungsbereich enthält, welcher komplementär zu den Sequenzen auf beiden Seiten der Spaltstelle ist, wo die Bindungsbereiche komplementär zu Sequenzen sind, welche ausgewählt werden aus der Gruppe bestehend aus: und
wobei "*" die Spaltstelle angibt.

5. Verfahren für den Aufbau eines Ribozyms zum Spalten eines menschlichen Papillomavirus-Transkripts gemäß Anspruch 4, wobei der Schritt des Aufbaus des besagten Haarnadel-Ribozyms das Miteinbinden einer Tetraschleife umfasst, welche die Sequenz besitzt, wie sie in der SEQ ID NO: 5 dargelegt ist.

6. Vektor, welcher eine DNA-Sequenz enthält, die für das besagte Ribozym kodiert ist, gemäß Anspruch 1, wobei die DNA operativ mit den Expressionskontrollsequenzen verbunden ist.

7. Wirtzelle, welche mit einem Vektor gemäß Anspruch 6 transformiert worden ist und welche zur Expression des besagten Ribozyms fähig ist, welches von dem besagten Vektor kodiert wird.

8. Ein in vitro Verfahren zum Aufspüren eines menschlichen Papillomavirus-16 (HPV-16) in einem menschlichen Gewebeextrakt, welches umfasst:
a. ein Aussetzen von RNA aus einer Probe aus menschlichem Gewebe gegen ein Ribozym gemäß Anspruch 1, welches sich an eine Nukleotidsequenz von HPV-16 RNA derart bindet, dass eine in der besagten Probe vorhandene HPV-16 RNA durch das besagte Ribozym gespalten wird;
b. ein Verstärken der cDNA unter Einsatz von Primern, welche komplementär sind zu: einem 5' Ende einer vollständigen Länge eines HPV-16-Transkripts, einem Fragment 5' der Ribozym-Spaltstelle der vollständigen Länge eines HPV-16-Transkripts, und einem Fragment 3' der Ribozym-Spaltstelle der vollständigen Länge eines HPV-16-Transkripts; und
c. ein Identifizieren der verstärkten DNA Fragmente derart, dass ein größeres DNA Fragment eine vollständige Länge eines HPV-16-Transkripts darstellt und ein kleineres DNA Fragment dasjenige Fragment darstellt, das sich aus dem Ribozym-Spalten des HPV-Transkripts mit der vollständigen Länge ergibt,
wobei, wenn HPV-16 RNA in der Probe vorhanden ist, eine Überlegenheit des kleineren Fragments über das größere Fragment identifiziert wird.

9. Verfahren gemäß Anspruch 8, wobei die von dem Ribozym gespaltene HPV-16 RNA ein E6 Transkript von HPV-16 ist.

10. Verfahren gemäß Anspruch 8, wobei das Ribozym die Sequenz von SEQ ID NO: 6 aufweist.

11. Verfahren gemäß Anspruch 8, wobei das menschliche Gewebe aus einem Cervixgewebe besteht.

12. Verfahren gemäß Anspruch 8, welches zusätzlich den Schritt der Erzeugung von cDNA aus der in der Probe vorhandenem RNA aufweist anschließend an den Schritt des Aussetzens und vor dem Schritt des Verstärkens.

13. Ribozym gemäß Anspruch 1 für die Verwendung bei der medizinischen Behandlung.

14. Ribozym gemäß Anspruch 13 für die Verwendung bei der Behandlung eines Cervixkarzinoms oder eines präkanzerösen Zustandes der Cervix.

15. Ribozym gemäß Anspruch 13, welches in einem auf einem Lipofektin beruhenden liposomalen Verabreichungssystem suspendiert ist.

16. Ribozym gemäß Anspruch 13 in Verbindung mit einem immunologischen Mittel oder mit einem chemotherapeutischen Mittel.

17. Ribozym gemäß Anspruch 13 in Verbindung mit LAK Zellen.

18. Ribozym gemäß Anspruch 13 in Verbindung mit Cisplatin.

## Revendications

1. Ribozyme en épingle à cheveux synthétique qui se lie à et clive un site cible dans un transcrit de papillomavirus humain, ledit site cible étant choisi dans le groupe constitué de sites cibles représentés par: et où « * » indique le site de clivage.

2. Ribozyme en épingle à cheveux synthétique selon la revendication 1, qui inclut une tétraboucle constituée de la SEQ ID NO: 5.

3. Ribozyme synthétique selon la revendication 1, constitué de la séquence complète telle que présentée dans la SEQ ID NO: 6.

4. Procédé pour la construction d'un ribozyme pour cliver un transcrit de papillomavirus humain comprenant l'étape de construction d'un ribozyme en épingle à cheveux, où un site de liaison sur le ribozyme inclut une séquence non complémentaire au site de clivage sur un papillomavirus humain et une région de liaison complémentaire aux séquences sur l'un ou l'autre côté du site de clivage où les régions de liaison sont complémentaires à des séquences choisies dans le groupe constitué de: et
où « * » indique le site de clivage.

5. Procédé pour la construction d'un ribozyme pour cliver un transcrit de papillomavirus humain selon la revendication 4, dans lequel l'étape de construction dudit ribozyme en épingle à cheveux inclut l'incorporation d'une tétraboucle comprenant la séquence présentée dans la SEQ ID NO: 5.

6. Vecteur comprenant une séquence d'ADN codant pour ledit ribozyme selon la revendication 1, l'ADN étant lié de manière fonctionnelle à des séquences de contrôle d'expression.

7. Cellule hôte transformée avec un vecteur selon la revendication 6 et qui est capable d'exprimer ledit ribozyme codé par ledit vecteur.

8. Procédé in vitro pour la détection d'un papillomavirus humain 16 (HPV-16) dans un extrait de tissu humain comprenant:
a. l'exposition d'un ARN provenant d'un échantillon d'un tissu humain à un ribozyme selon la revendication 1, qui se lie à une séquence de nucléotides provenant d'un ARN de HPV-16, de sorte que l'ARN de HPV-16 présent dans ledit échantillon est clivé par ledit ribozyme;
b. l'amplification de l'ADNc en employant des amorces complémentaires à: une extrémité 5' d'un transcrit de HPV-16 pleine longueur, un fragment 5' du site de clivage par le ribozyme du transcrit de HPV-16 pleine longueur et un fragment 3' du site de clivage par le ribozyme du transcrit de HPV-16 pleine longueur; et
c. l'identification des fragments d'ADN amplifiés, de sorte qu'un fragment d'ADN plus grand représente un transcrit de HPV-16 pleine longueur et un fragment d'ADN de plus petite taille représente le fragment résultant d'un clivage par le ribozyme du transcrit de HPV pleine longueur,
dans lequel, si l'ARN de HPV-16 est présent dans l'échantillon, une prépondérance du fragment plus petit est identifiée relativement au fragment plus grand.

9. Procédé selon la revendication 8, dans lequel l'ARN de HPV-16 clivé par le ribozyme est un transcrit E6 de HPV-16.

10. Procédé selon la revendication 8, dans lequel le ribozyme comprend la séquence de la SEQ ID NO: 6.

11. Procédé selon la revendication 8, dans lequel le tissu humain est un tissu du col de l'utérus.

12. Procédé selon la revendication 8, comprenant en outre l'étape de production d'un ADNc à partir de l'ARN présent dans l'échantillon après l'étape d'exposition et avant l'étape d'amplification.

13. Ribozyme selon la revendication 1, pour une utilisation dans un traitement médical.

14. Ribozyme selon la revendication 13, pour une utilisation dans le traitement d'un cancer du col de l'utérus ou d'un état précancéreux du col.

15. Ribozyme selon la revendication 13, en suspension dans un système d'administration liposomale à base de lipofectine.

16. Ribozyme selon la revendication 13, en combinaison avec un agent immunologique ou un agent chimiothérapeutique.

17. Ribozyme selon la revendication 13, en combinaison avec des cellules LAK.

18. Ribozyme selon la revendication 13, en combinaison avec du cisplatine.


### SEQUENCE LISTING

<110> THE GOVERNMENT OF THE UNITED STATES OF AMERICA, AS REPRESENTED BY THE SECRETARY, DEPARTMENT OF HEALTH AND HUMAN SERVICES, AND NORTHERN ILLINOIS UNIVERSITY
<120> HUMAN PAPILLOMA VIRUS INHIBITION BY A HAIRPIN RIBOZYME
<130> NIH113.001QEP
<140> 95920437.1
   <141> 1995-05-15
<160> 18
<170> FastSEQ for Windows Version 4.0
<21C> 1
   <211> 7902
   <212> DNA
   <213> Human papillomavirus, strain HPV16
<400> 1
<210> 2
   <211> 15
   <212> RNA
   <213> Human papillomavirus
<400> 2
   acuguguccu gaaga 15
<210> 3
   <211> 16
   <212> RNA
   <213> Human papillomavirus
<400> 3
   acuguguccu gaagaa 16
<210> 4
   <211> 17
   <212> RNA
   <213> Human papillomavirus
<400> 4
   acuguguccu gaagaaa 17
<210> 5
   <211> 12
   <212> RNA
   <213> Human papillomavirus
<400> 5
   ggacuucggu cc 12
<210> 6
   <211> 61
   <212> RNA
   <213> Artificial Sequence
<220>
   <223> ribozyme
<400> 6
   uucuucagag aacaguacca gagaaacaca cggacuucgg uccgugguau auuaccuggu 60
   a 61
<210> 7
   <211> 14
   <212> RNA
   <213> Human papillomavirus
<400> 7
   uaacugucaa aagc 14
<210> 8
   <211> 15
   <212> RNA
   <213> Human papillomavirus
<400> 8
   uaacugucaa aagcc 15
<210> 9
   <211> 16
   <212> RNA
   <213> Human papillomavirus
<400> 9
   uaacugucaa aagcca 16
<210> 10
   <211> 17
   <212> RNA
   <213> Human papillomavirus
<400> 10
   uaacugucaa aagccac 17
<210> 11
   <211> 60
   <212> RNA
   <213> Artificial Sequence
<220>
   <223> ribozyme
<400> 11
   ggcuuuuaga aguuaaccag agaaacacac ggacuucggu ccgugguaua uuaccuggua 60
<210> 12
   <211> 14
   <212> DNA
   <213> Human papillomavirus
<400> 12
   ttatggtttc tgag 14
<210> 13
   <211> 27
   <212> DNA
   <213> Human papillomavirus
<400> 13
   ccctctagag gcacacaatt cctagtg 27
<210> 14
   <211> 18
   <212> DNA
   <213> Human papillomavirus
<400> 14
   cacgtagaga aacccagc 18
<210> 15
   <211> 18
   <212> DNA
   <213> Human papillomavirus
<400> 15
   cagcaataca acaaaccg 18
<210> 16
   <211> 12
   <212> RNA
   <213> Artificial Sequence
<220>
   <223> inverted sequence
<400> 16
   ccuguucgca gg 12
<210> 17
   <211> 28
   <212> DNA
   <213> Artificial Sequence
<220>
   <223> primer
<221> misc_feature
   <222> (0) ... (0)
   <223> n=a, t, c, or g
<400> 17
   nnnaagcttc tgcaatgttt caggaccc 28
<210> 18
   <211> 29
   <212> DNA
   <213> Artificial Sequence
<220>
   <223> primer
<221> misc_feature
   <222> (0)...(0)
   <223> n=a,t,c, or g
<400> 18
   nnnggatccc cattggtacc tgcaggatc 29